# EUROPÄISCHE PATENTSCHRIFT

(11) **EP 2 126 065 B1**
(45) Veröffentlichungstag und Bekanntmachung des Hinweises auf die Patenterteilung: **19.06.2013**
(21) Anmeldenummer: 08717626.9
(22) Anmeldetag: 11.03.2008
(51) Int. Cl.: C12N 9/18

(54) **ISOFORMEN DER SCHWEINELEBER ESTERASE**
ISOFORMS OF PIG LIVER ESTERASE
ISOFORMES DE L'ESTÉRASE DE FOIE DE PORC

(30) Priorität: 23.03.2007 DE 102007014742
(43) Veröffentlichungstag der Anmeldung: 02.12.2009
(73) Patentinhaber: Enzymicals AG, 17489 Greifswald (DE)
(72) Erfinder: BORNSCHEUER, Uwe T., 17489 Greifswald (DE); HUMMEL, Anke, 52445 Titz (DE); BÖTTCHER, Dominique, 17039 Sponholz (DE); BRÜSEHABER, Elke, 17491 Greifswald (DE); DODERER, Kai, 63110 Rodgau (DE); TRAUTHWEIN, Harald, 68642 Bürstadt (DE)
(74) Vertreter: Vossius & Partner
(86) Internationale Anmeldenummer: PCT/EP2008/052880
(87) Internationale Veröffentlichungsnummer: WO 2008/116745

(56) Entgegenhaltungen:
- WO-A-2004/055177
- MUSIDLOWSKA-PERSSON A ET AL: "Site directed mutagenesis of recombinant pig liver esterase yields mutants with altered enantioselectivity" TETRAHEDRON: ASYMMETRY, ELSEVIER SCIENCE PUBLISHERS, AMSTERDAM, NL, Bd. 14, Nr. 10, 16. Mai 2003 (2003-05-16), Seiten 1341-1344, XP004439169 ISSN: 0957-4166
- DOMINIQUE BÖTTCHER ET AL: "Functional expression of the [gamma]-isoenzyme of pig liver carboxyl esterase in Escherichia coli" APPLIED MICROBIOLOGY AND BIOTECHNOLOGY, SPRINGER-VERLAG, BE, Bd. 73, Nr. 6, 8. September 2006 (2006-09-08), Seiten 1282-1289, XP019472507 ISSN: 1432-0614
- BRÜSEHABER E ET AL: "Identification of pig liver esterase variants by tandem mass spectroscopy analysis and their characterization" APPLIED MICROBIOLOGY AND BIOTECHNOLOGY, SPRINGER-VERLAG, BE, Bd. 76, Nr. 4, 26. Juni 2007 (2007-06-26), Seiten 853-859, XP019538722 ISSN: 1432-0614

### SEQUENCE LISTING

<110> Degussa GmbH
<120> PLE-Isoformen
<130> 200700099
<160> 12
<170> PatentIn version 3.3
<210> 1
   <211> 1698
   <212> DNA
   <213> Pig liver
<220>
   <221> CDS
   <222> (1)..(1698)
<400> 1
   atg ggc agc agc cat cat cat cat cat cac agc agc ggc ctg gtg ccg       48
   Met Gly Ser Ser His His His His His His Ser Ser Gly Leu Val Pro
   1               5                   10                  15
   cgc ggc agc cat atg ggg cag cca gcc tcg ccg cct gtt gtg gac act       96
   Arg Gly Ser His Met Gly Gln Pro Ala Ser Pro Pro Val Val Asp Thr
               20                  25                  30
   gcc cag ggc cga gtc ctg ggg aag tac gtc agc tta gaa ggc ctg gca      144
   Ala Gln Gly Arg Val Leu Gly Lys Tyr Val Ser Leu Glu Gly Leu Ala
           35                  40                  45
   cag ccg gtg gcc gtc ttc ctg gga gtc cct ttt gcc aag ccc cct ctc      192
   Gln Pro Val Ala Val Phe Leu Gly Val Pro Phe Ala Lys Pro Pro Leu
       50                  55                  60
   gga tcc ttg agg ttt gct ccg ccg cag cct gca gaa cca tgg agc ttc      240
   Gly Ser Leu Arg Phe Ala Pro Pro Gln Pro Ala Glu Pro Trp Ser Phe
   65                  70                  75                  80
   gtg aag aac acc acc tcc tac cct ccc atg tgc tgc cag gac cca gta      288
   Val Lys Asn Thr Thr Ser Tyr Pro Pro Met Cys Cys Gln Asp Pro Val
                   85                  90                  95
   gtg gag cag atg acc tca gat cta ttt acc aac gga aag gag agg ctc      336
   Val Glu Gln Met Thr Ser Asp Leu Phe Thr Asn Gly Lys Glu Arg Leu
               100                 105                 110
   act ctg gag ttt tct gaa gac tgt ctc tac cta aat att tac acc cct      384
   Thr Leu Glu Phe Ser Glu Asp Cys Leu Tyr Leu Asn Ile Tyr Thr Pro
           115                 120                 125
   gct gac ctg aca aag agg ggc aga ctg ccg gtg atg gtg tgg atc cac      432
   Ala Asp Leu Thr Lys Arg Gly Arg Leu Pro Val Met Val Trp Ile His
       130                 135                 140
   gga gga ggc ctg gtg ttg ggc ggg gca cca atg tat gat ggg gtg gtg      480
   Gly Gly Gly Leu Val Leu Gly Gly Ala Pro Met Tyr Asp Gly Val Val
   145                 150                 155                 160
   ctt gct gcg cat gaa aac gtg gtg gtg gtg gcc atc cag tac cgc ctg      528
   Leu Ala Ala His Glu Asn Val Val Val Val Ala Ile Gln Tyr Arg Leu
                   165                 170                 175
   ggc atc tgg gga ttc ttc agc aca ggg gat gaa cac agc cgg ggc aac      576
   Gly Ile Trp Gly Phe Phe Ser Thr Gly Asp Glu His Ser Arg Gly Asn
                   180                 185                 190
   tgg ggt cac ttg gac cag gtg gcc gca ctg cac tgg gtc cag gag aac      624
   Trp Gly His Leu Asp Gln Val Ala Ala Leu His Trp Val Gln Glu Asn
           195                 200                 205
   atc gcc aac ttt gga ggc gac cca ggc tct gtg acc atc ttt gga gag      672
   Ile Ala Asn Phe Gly Gly Asp Pro Gly Ser Val Thr Ile Phe Gly Glu
       210                 215                 220
   tca gca gga ggg gaa agt gtc tct gtt ctg gtg ttg tct ccc ttg gcc      720
   Ser Ala Gly Gly Glu Ser Val Ser Val Leu Val Leu Ser Pro Leu Ala
   225                 230                 235                 240
   aag aac ctc ttc cac cgg gcc atc tct gag agt ggc gtg gcc ctc act      768
   Lys Asn Leu Phe His Arg Ala Ile Ser Glu Ser Gly Val Ala Leu Thr
                   245                 250                 255
   gtt gcc ctg gtc agg aag gac atg aag gct gca gct aag caa att gct      816
   Val Ala Leu Val Arg Lys Asp Met Lys Ala Ala Ala Lys Gln Ile Ala
               260                 265                 270
   gtc ctt gct ggg tgt aaa acc acc acc tcg gct gtc ttt gtt cac tgc      864
   Val Leu Ala Gly Cys Lys Thr Thr Thr Ser Ala Val Phe Val His Cys
           275                 280                 285
   ctg cgc cag aag tcg gag gac gag ctc ttg gac tta acg ctg aag atg      912
   Leu Arg Gln Lys Ser Glu Asp Glu Leu Leu Asp Leu Thr Leu Lys Met
       290                 295                 300
   aaa ttt tta act ctt gat ttt cat gga gac caa aga gag agc cat ccc      960
   Lys Phe Leu Thr Leu Asp Phe His Gly Asp Gln Arg Glu Ser His Pro
   305                 310                 315                 320
   ttc ctg ccc act gtg gtg gat gga gtg ctg ctg ccc aag atg cct gaa     1008
   Phe Leu Pro Thr Val Val Asp Gly Val Leu Leu Pro Lys Met Pro Glu
                   325                 330                 335
   gag att ctg gct gag aag gat ttc aac act gtc ccc tac atc gtg gga     1056
   Glu Ile Leu Ala Glu Lys Asp Phe Asn Thr Val Pro Tyr Ile Val Gly
               340                 345                 350
   atc aac aag caa gag ttt ggc tgg ctt ctg cca acg atg atg ggc ttc     1104
   Ile Asn Lys Gln Glu Phe Gly Trp Leu Leu Pro Thr Met Met Gly Phe
           355                 360                 365
   ccc ctc tct gaa ggc aag ctg gac cag aag acg gcc acg tca ctc ctg     1152
   Pro Leu Ser Glu Gly Lys Leu Asp Gln Lys Thr Ala Thr Ser Leu Leu
       370                 375                 380
   tgg aag tcc tac ccc atc gct aac atc cct gag gaa ctg act cca gtg     1200
   Trp Lys Ser Tyr Pro Ile Ala Asn Ile Pro Glu Glu Leu Thr Pro Val
   385                 390                 395                 400
   gcc act gac aag tat ttg ggg ggg aca gac gac ccc gtc aaa aag aaa     1248
   Ala Thr Asp Lys Tyr Leu Gly Gly Thr Asp Asp Pro Val Lys Lys Lys
                   405                 410                 415
   gac ctg ttc ctg gac ttg atg ggg gat gtg gtg ttt ggt gtc cca tct     1296
   Asp Leu Phe Leu Asp Leu Met Gly Asp Val Val Phe Gly Val Pro Ser
               420                 425                 430
   gtg acg gtg gcc cgt caa cac aga gat gca gga gcc ccc acc tac atg     1344
   Val Thr Val Ala Arg Gln His Arg Asp Ala Gly Ala Pro Thr Tyr Met
           435                 440                 445
   tat gag ttt cag tat cgc cca agc ttc tca tcg gac aag aaa ccc aag     1392
   Tyr Glu Phe Gln Tyr Arg Pro Ser Phe Ser Ser Asp Lys Lys Pro Lys
       450                 455                 460
   acg gtg atc ggg gac cac ggg gat gag atc ttc tcc gtc ttt ggt ttt        1440
   Thr Val Ile Gly Asp His Gly Asp Glu Ile Phe Ser Val Phe Gly Phe
   465                 470                 475                 480
   cca ctg tta aaa ggc gat gcc cca gaa gag gag gtc agt ctc agc aag        1488
   Pro Leu Leu Lys Gly Asp Ala Pro Glu Glu Glu Val Ser Leu Ser Lys
                   485                 490                 495
   acg gtg atg aaa ttc tgg gcc aac ttt gct cgc agt ggg aac ccc aat        1536
   Thr Val Met Lys Phe Trp Ala Asn Phe Ala Arg Ser Gly Asn Pro Asn
               500                 505                 510
   ggg gag ggg ctg ccc cat tgg ccg atg tac gac cag gaa gaa ggg tac        1584
   Gly Glu Gly Leu Pro His Trp Pro Met Tyr Asp Gln Glu Glu Gly Tyr
           515                 520                 525
   ctt cag atc ggc gtc aac acc cag gca gcc aag agg ctg aaa ggt gaa        1632
   Leu Gln Ile Gly Val Asn Thr Gln Ala Ala Lys Arg Leu Lys Gly Glu
       530                 535                 540
   gaa gtg gcc ttc tgg aac gat ctc ctg tcc aag gag gca gca aag aag        1680
   Glu Val Ala Phe Trp Asn Asp Leu Leu Ser Lys Glu Ala Ala Lys Lys
   545                 550                 555                 560
   cca ccc aag ata aag tga                                                1698
   Pro Pro Lys Ile Lys
                   565
<210> 2
   <211> 565
   <212> PRT
   <213> Pig liver
<400> 2
   Met Gly Ser Ser His His His His His His Ser Ser Gly Leu Val Pro
   1               5                   10                  15
   Arg Gly Ser His Met Gly Gln Pro Ala Ser Pro Pro Val Val Asp Thr
               20                  25                  30
   Ala Gln Gly Arg Val Leu Gly Lys Tyr Val Ser Leu Glu Gly Leu Ala
           35                  40                  45
   Gln Pro Val Ala Val Phe Leu Gly Val Pro Phe Ala Lys Pro Pro Leu
       50                  55                  60
   Gly Ser Leu Arg Phe Ala Pro Pro Gln Pro Ala Glu Pro Trp Ser Phe
   65                  70                  75                  80
   Val Lys Asn Thr Thr Ser Tyr Pro Pro Met Cys Cys Gln Asp Pro Val
                   85                  90                  95
   Val Glu Gln Met Thr Ser Asp Leu Phe Thr Asn Gly Lys Glu Arg Leu
               100                 105                 110
   Thr Leu Glu Phe Ser Glu Asp Cys Leu Tyr Leu Asn Ile Tyr Thr Pro
           115                 120                 125
   Ala Asp Leu Thr Lys Arg Gly Arg Leu Pro Val Met Val Trp Ile His
       130                 135                 140
   Gly Gly Gly Leu Val Leu Gly Gly Ala Pro Met Tyr Asp Gly Val Val
   145                 150                 155                 160
   Leu Ala Ala His Glu Asn Val Val Val Val Ala Ile Gln Tyr Arg Leu
                   165                 170                 175
   Gly Ile Trp Gly Phe Phe Ser Thr Gly Asp Glu His Ser Arg Gly Asn
               180                 185                 190
   Trp Gly His Leu Asp Gln Val Ala Ala Leu His Trp Val Gln Glu Asn
           195                 200                 205
   Ile Ala Asn Phe Gly Gly Asp Pro Gly Ser Val Thr Ile Phe Gly Glu
       210                 215                 220
   Ser Ala Gly Gly Glu Ser Val Ser Val Leu Val Leu Ser Pro Leu Ala
   225                 230                 235                 240
   Lys Asn Leu Phe His Arg Ala Ile Ser Glu Ser Gly Val Ala Leu Thr
                   245                 250                 255
   Val Ala Leu Val Arg Lys Asp Met Lys Ala Ala Ala Lys Gln Ile Ala
               260                 265                 270
   Val Leu Ala Gly Cys Lys Thr Thr Thr Ser Ala Val Phe Val His Cys
           275                 280                 285
   Leu Arg Gln Lys Ser Glu Asp Glu Leu Leu Asp Leu Thr Leu Lys Met
       290                 295                 300
   Lys Phe Leu Thr Leu Asp Phe His Gly Asp Gln Arg Glu Ser His Pro
   305                 310                 315                 320
   Phe Leu Pro Thr Val Val Asp Gly Val Leu Leu Pro Lys Met Pro Glu
                   325                 330                 335
   Glu Ile Leu Ala Glu Lys Asp Phe Asn Thr Val Pro Tyr Ile Val Gly
               340                 345                 350
   Ile Asn Lys Gln Glu Phe Gly Trp Leu Leu Pro Thr Met Met Gly Phe
           355                 360                 365
   Pro Leu Ser Glu Gly Lys Leu Asp Gln Lys Thr Ala Thr Ser Leu Leu
       370                 375                 380
   Trp Lys Ser Tyr Pro Ile Ala Asn Ile Pro Glu Glu Leu Thr Pro Val
   385                 390                 395                 400
   Ala Thr Asp Lys Tyr Leu Gly Gly Thr Asp Asp Pro Val Lys Lys Lys
                   405                 410                 415
   Asp Leu Phe Leu Asp Leu Met Gly Asp Val Val Phe Gly Val Pro Ser
               420                 425                 430
   Val Thr Val Ala Arg Gln His Arg Asp Ala Gly Ala Pro Thr Tyr Met
           435                 440                 445
   Tyr Glu Phe Gln Tyr Arg Pro Ser Phe Ser Ser Asp Lys Lys Pro Lys
       450                 455                 460
   Thr Val Ile Gly Asp His Gly Asp Glu Ile Phe Ser Val Phe Gly Phe
   465                 470                 475                 480
   Pro Leu Leu Lys Gly Asp Ala Pro Glu Glu Glu Val Ser Leu Ser Lys
                   485                 490                 495
   Thr Val Met Lys Phe Trp Ala Asn Phe Ala Arg Ser Gly Asn Pro Asn
               500                 505                 510
   Gly Glu Gly Leu Pro His Trp Pro Met Tyr Asp Gln Glu Glu Gly Tyr
           515                 520                 525
   Leu Gln Ile Gly Val Asn Thr Gln Ala Ala Lys Arg Leu Lys Gly Glu
       530                 535                 540
   Glu Val Ala Phe Trp Asn Asp Leu Leu Ser Lys Glu Ala Ala Lys Lys
   545                 550                 555                 560
   Pro Pro Lys Ile Lys
                   565
<210> 3
   <211> 1698
   <212> DNA
   <213> Artificial
<220>
   <223> Mutante der PLE
<220>
   <221> CDS
   <222> (1)..(1698)
<400> 3
   atg ggc agc agc cat cat cat cat cat cac agc agc ggc ctg gtg ccg       48
   Met Gly Ser Ser His His His His His His Ser Ser Gly Leu Val Pro
   1               5                   10                  15
   cgc ggc agc cat atg ggg cag cca gcc tcg ccg cct gtt gtg gac act       96
   Arg Gly Ser His Met Gly Gln Pro Ala Ser Pro Pro Val Val Asp Thr
               20                  25                  30
   gcc cag ggc cga gtc ctg ggg aag tac gtc agc tta gaa ggc ctg gca      144
   Ala Gln Gly Arg Val Leu Gly Lys Tyr Val Ser Leu Glu Gly Leu Ala
           35                  40                  45
   cag ccg gtg gcc gtc ttc ctg gga gtc cct ttt gcc aag ccc cct ctc      192
   Gln Pro Val Ala Val Phe Leu Gly Val Pro Phe Ala Lys Pro Pro Leu
       50                  55                  60
   gga tcc ttg agg ttt gct ccg ccg cag cct gca gaa cca tgg agc ttc      240
   Gly Ser Leu Arg Phe Ala Pro Pro Gln Pro Ala Glu Pro Trp Ser Phe
   65                  70                  75                  80
   gtg aag aac acc acc tcc tac cct ccc atg tgc tgc cag gac cca gta      288
   Val Lys Asn Thr Thr Ser Tyr Pro Pro Met Cys Cys Gln Asp Pro Val
                   85                  90                  95
   gtg gag cag atg acc tca gat cta ttt acc aac gga aag gag agg ctc      336
   Val Glu Gln Met Thr Ser Asp Leu Phe Thr Asn Gly Lys Glu Arg Leu
               100                 105                 110
   act ctg gag ttt tct gaa gac tgt ctc tac cta aat att tac acc cct      384
   Thr Leu Glu Phe Ser Glu Asp Cys Leu Tyr Leu Asn Ile Tyr Thr Pro
           115                 120                 125
   gct gac ctg aca aag agg ggc aga ctg ccg gtg atg gtg tgg atc cac      432
   Ala Asp Leu Thr Lys Arg Gly Arg Leu Pro Val Met Val Trp Ile His
       130                 135                 140
   gga gga ggc ctg gtg ttg ggc ggg gca cca atg tat gat ggg gtg gtg      480
   Gly Gly Gly Leu Val Leu Gly Gly Ala Pro Met Tyr Asp Gly Val Val
   145                 150                 155                 160
   ctt gct gcg cat gaa aac gtg gtg gtg gtg gcc atc cag tac cgc ctg      528
   Leu Ala Ala His Glu Asn Val Val Val Val Ala Ile Gln Tyr Arg Leu
                   165                 170                 175
   ggc atc tgg gga ttc ttc agc aca ggg gat gaa cac agc cgg ggc aac      576
   Gly Ile Trp Gly Phe Phe Ser Thr Gly Asp Glu His Ser Arg Gly Asn
               180                 185                 190
   tgg ggt cac ttg gac cag gtg gcc gca ctg cac tgg gtc cag gag aac      624
   Trp Gly His Leu Asp Gln Val Ala Ala Leu His Trp Val Gln Glu Asn
           195                 200                 205
   atc gcc aac ttt gga ggc gac cca ggc tct gtg acc atc ttt gga gag      672
   Ile Ala Asn Phe Gly Gly Asp Pro Gly Ser Val Thr Ile Phe Gly Glu
       210                 215                 220
   tca gca gga ggg gaa agt gtc tct gtt ctg gtg ttg tct ccc ttg gcc      720
   Ser Ala Gly Gly Glu Ser Val Ser Val Leu Val Leu Ser Pro Leu Ala
   225                 230                 235                 240
   aag aac ctc ttc cac cgg gcc atc tct gag agt ggc gtg gcc ctc act      768
   Lys Asn Leu Phe His Arg Ala Ile Ser Glu Ser Gly Val Ala Leu Thr
                   245                 250                 255
   gtt gcc ctg gtc agg aag gac atg aag gct gca gct aag caa att gct      816
   Val Ala Leu Val Arg Lys Asp Met Lys Ala Ala Ala Lys Gln Ile Ala
               260                 265                 270
   gtc ctt gct ggg tgt aaa acc acc acc tcg gct gtc ttt gtt cac tgc      864
   Val Leu Ala Gly Cys Lys Thr Thr Thr Ser Ala Val Phe Val His Cys
           275                 280                 285
   ctg cgc cag aag tcg gag gac gag ctc ttg gac tta acg ctg aag atg      912
   Leu Arg Gln Lys Ser Glu Asp Glu Leu Leu Asp Leu Thr Leu Lys Met
       290                 295                 300
   aaa ttt tta act ctt gat ttt cat gga gac caa aga gag agc cat ccc      960
   Lys Phe Leu Thr Leu Asp Phe His Gly Asp Gln Arg Glu Ser His Pro
   305                 310                 315                 320
   ttc ctg ccc act gtg gtg gat gga gtg ctg ctg ccc aag atg cct gaa     1008
   Phe Leu Pro Thr Val Val Asp Gly Val Leu Leu Pro Lys Met Pro Glu
   325 330 335
   gag att ctg gct gag aag gat ttc aac act gtc ccc tac atc gtg gga 1056
   Glu Ile Leu Ala Glu Lys Asp Phe Asn Thr Val Pro Tyr Ile Val Gly
   340 345 350
   atc aac aag caa gag ttt ggc tgg ctt ctg cca acg atg atg ggc ttc 1104
   Ile Asn Lys Gln Glu Phe Gly Trp Leu Leu Pro Thr Met Met Gly Phe
   355 360 365
   ccc ctc tct gaa ggc aag ctg gac cag aag acg gcc acg tca ctc ctg 1152
   Pro Leu Ser Glu Gly Lys Leu Asp Gln Lys Thr Ala Thr Ser Leu Leu
   370 375 380
   tgg aag tcc tac ccc atc gct aac atc cct gag gaa ctg act cca gtg 1200
   Trp Lys Ser Tyr Pro Ile Ala Asn Ile Pro Glu Glu Leu Thr Pro Val
   385 390 395 400
   gcc act gac aag tat ttg ggg ggg aca gac gac ccc gtc aaa aag aaa 1248
   Ala Thr Asp Lys Tyr Leu Gly Gly Thr Asp Asp Pro Val Lys Lys Lys
   405 410 415
   gac ctg ttc ctg gac ttg atg ggg gat gtg gtg ttt ggt gtc cca tct 1296
   Asp Leu Phe Leu Asp Leu Met Gly Asp Val Val Phe Gly Val Pro Ser
   420 425 430
   gtg acg gtg gcc cgt caa cac aga gat gca gga gcc ccc acc tac atg 1344
   Val Thr Val Ala Arg Gln His Arg Asp Ala Gly Ala Pro Thr Tyr Met
   435 440 445
   tat gag ttt cag tat cgc cca agc ttc tca tcg gac aag aaa ccc aag 1392
   Tyr Glu Phe Gln Tyr Arg Pro Ser Phe Ser Ser Asp Lys Lys Pro Lys
   450 455 460
   acg gtg atc ggg gac cac ggg gat gag atc ttc tcc gtc ttt ggg gct 1440
   Thr Val Ile Gly Asp His Gly Asp Glu Ile Phe Ser Val Phe Gly Ala
   465 470 475 480
   cca ttt tta aga ggc gat gcc cca gaa gag gag gtc agt ctc agc aag 1488
   Pro Phe Leu Arg Gly Asp Ala Pro Glu Glu Glu Val Ser Leu Ser Lys
   485 490 495
   acg gtg atg aaa ttc tgg gcc aac ttt gct cgc agt ggg aac ccc aat 1536
   Thr Val Met Lys Phe Trp Ala Asn Phe Ala Arg Ser Gly Asn Pro Asn
   500 505 510
   ggg gag ggg ctg ccc cat tgg ccg atg tac gac cag gaa gaa ggg tac 1584
   Gly Glu Gly Leu Pro His Trp Pro Met Tyr Asp Gln Glu Glu Gly Tyr
   515 520 525
   ctt cag atc ggc gtc aac acc cag gca gcc aag agg ctg aaa ggt gaa 1632
   Leu Gln Ile Gly Val Asn Thr Gln Ala Ala Lys Arg Leu Lys Gly Glu
   530 535 540
   gaa gtg gcc ttc tgg aac gat ctc ctg tcc aag gag gca gca aag aag 1680
   Glu Val Ala Phe Trp Asn Asp Leu Leu Ser Lys Glu Ala Ala Lys Lys
   545 550 555 560
   cca ccc aag ata aag tga 1698
   Pro Pro Lys Ile Lys
   565
<210> 4
   <211> 565
   <212> PRT
   <213> Artificial
<220>
   <223> Synthetic Construct
<400> 4
   Met Gly Ser Ser His His His His His His Ser Ser Gly Leu Val Pro
   1               5                   10                  15
   Arg Gly Ser His Met Gly Gln Pro Ala Ser Pro Pro Val Val Asp Thr
               20                  25                  30
   Ala Gln Gly Arg Val Leu Gly Lys Tyr Val Ser Leu Glu Gly Leu Ala
           35                  40                  45
   Gln Pro Val Ala Val Phe Leu Gly Val Pro Phe Ala Lys Pro Pro Leu
       50                  55                  60
   Gly Ser Leu Arg Phe Ala Pro Pro Gln Pro Ala Glu Pro Trp Ser Phe
   65                  70                  75                  80
   Val Lys Asn Thr Thr Ser Tyr Pro Pro Met Cys Cys Gln Asp Pro Val
                   85                  90                  95
   Val Glu Gln Met Thr Ser Asp Leu Phe Thr Asn Gly Lys Glu Arg Leu
               100                 105                 110
   Thr Leu Glu Phe Ser Glu Asp Cys Leu Tyr Leu Asn Ile Tyr Thr Pro
           115                 120                 125
   Ala Asp Leu Thr Lys Arg Gly Arg Leu Pro Val Met Val Trp Ile His
       130                 135                 140
   Gly Gly Gly Leu Val Leu Gly Gly Ala Pro Met Tyr Asp Gly Val Val
   145                 150                 155                 160
   Leu Ala Ala His Glu Asn Val Val Val Val Ala Ile Gln Tyr Arg Leu
                   165                 170                 175
   Gly Ile Trp Gly Phe Phe Ser Thr Gly Asp Glu His Ser Arg Gly Asn
               180                 185                 190
   Trp Gly His Leu Asp Gln Val Ala Ala Leu His Trp Val Gln Glu Asn
           195                 200                 205
   Ile Ala Asn Phe Gly Gly Asp Pro Gly Ser Val Thr Ile Phe Gly Glu
       210                 215                 220
   Ser Ala Gly Gly Glu Ser Val Ser Val Leu Val Leu Ser Pro Leu Ala
   225                 230                 235                 240
   Lys Asn Leu Phe His Arg Ala Ile Ser Glu Ser Gly Val Ala Leu Thr
                   245                 250                 255
   Val Ala Leu Val Arg Lys Asp Met Lys Ala Ala Ala Lys Gln Ile Ala
               260                 265                 270
   Val Leu Ala Gly Cys Lys Thr Thr Thr Ser Ala Val Phe Val His Cys
           275                 280                 285
   Leu Arg Gln Lys Ser Glu Asp Glu Leu Leu Asp Leu Thr Leu Lys Met
       290                 295                 300
   Lys Phe Leu Thr Leu Asp Phe His Gly Asp Gln Arg Glu Ser His Pro
   305                 310                 315                 320
   Phe Leu Pro Thr Val Val Asp Gly Val Leu Leu Pro Lys Met Pro Glu
                   325                 330                 335
   Glu Ile Leu Ala Glu Lys Asp Phe Asn Thr Val Pro Tyr Ile Val Gly
               340                 345                 350
   Ile Asn Lys Gln Glu Phe Gly Trp Leu Leu Pro Thr Met Met Gly Phe
           355                 360                 365
   Pro Leu Ser Glu Gly Lys Leu Asp Gln Lys Thr Ala Thr Ser Leu Leu
       370                 375                 380
   Trp Lys Ser Tyr Pro Ile Ala Asn Ile Pro Glu Glu Leu Thr Pro Val
   385                 390                 395                 400
   Ala Thr Asp Lys Tyr Leu Gly Gly Thr Asp Asp Pro Val Lys Lys Lys
                   405                 410                 415
   Asp Leu Phe Leu Asp Leu Met Gly Asp Val Val Phe Gly Val Pro Ser
               420                 425                 430
   Val Thr Val Ala Arg Gln His Arg Asp Ala Gly Ala Pro Thr Tyr Met
           435                 440                 445
   Tyr Glu Phe Gln Tyr Arg Pro Ser Phe Ser Ser Asp Lys Lys Pro Lys
       450                 455                 460
   Thr Val Ile Gly Asp His Gly Asp Glu Ile Phe Ser Val Phe Gly Ala
   465                 470                 475                 480
   Pro Phe Leu Arg Gly Asp Ala Pro Glu Glu Glu Val Ser Leu Ser Lys
                   485                 490                 495
   Thr Val Met Lys Phe Trp Ala Asn Phe Ala Arg Ser Gly Asn Pro Asn
               500                 505                 510
   Gly Glu Gly Leu Pro His Trp Pro Met Tyr Asp Gln Glu Glu Gly Tyr
           515                 520                 525
   Leu Gln Ile Gly Val Asn Thr Gln Ala Ala Lys Arg Leu Lys Gly Glu
   530 535 540
   Glu Val Ala Phe Trp Asn Asp Leu Leu Ser Lys Glu Ala Ala Lys Lys
   545 550 555 560
   Pro Pro Lys Ile Lys
   565
<210> 5
   <211> 1698
   <212> DNA
   <213> Artificial
<220>
   <223> Mutante der PLE
<220>
   <221> CDS
   <222> (1)..(1698)
<400> 5
   atg ggc agc agc cat cat cat cat cat cac agc agc ggc ctg gtg ccg 48
   Met Gly Ser Ser His His His His His His Ser Ser Gly Leu Val Pro
   1 5 10 15
   cgc ggc agc cat atg ggg cag cca gcc tcg ccg cct gtt gtg gac act 96
   Arg Gly Ser His Met Gly Gln Pro Ala Ser Pro Pro Val Val Asp Thr
   20 25 30
   gcc cag ggc cga gtc ctg ggg aag tac gtc agc tta gaa ggc ctg gca 144
   Ala Gln Gly Arg Val Leu Gly Lys Tyr Val Ser Leu Glu Gly Leu Ala
   35 40 45
   cag ccg gtg gcc gtc ttc ctg gga gtc cct ttt gcc aag ccc cct ctc 192
   Gln Pro Val Ala Val Phe Leu Gly Val Pro Phe Ala Lys Pro Pro Leu
   50 55 60
   gga tcc ttg agg ttt gct ccg ccg cag cct gca gaa cca tgg agc ttc 240
   Gly Ser Leu Arg Phe Ala Pro Pro Gln Pro Ala Glu Pro Trp Ser Phe
   65 70 75 80
   gtg aag aac acc acc tcc tac cct ccc atg tgc tgc cag gac cca gta 288
   Val Lys Asn Thr Thr Ser Tyr Pro Pro Met Cys Cys Gln Asp Pro Val
   85 90 95
   gcg ggg cag atg acc tca gat cta ttt acc aac aga aag gag agg ctc 336
   Ala Gly Gln Met Thr Ser Asp Leu Phe Thr Asn Arg Lys Glu Arg Leu
   100 105 110
   att ccg gag ttt tct gaa gac tgt ctc tac cta aat att tac acc cct 384
   Ile Pro Glu Phe Ser Glu Asp Cys Leu Tyr Leu Asn Ile Tyr Thr Pro
   115 120 125
   gct gac ctg aca aag agg ggc aga ctg ccg gtg atg gtg tgg atc cac 432
   Ala Asp Leu Thr Lys Arg Gly Arg Leu Pro Val Met Val Trp Ile His
   130 135 140
   gga gga ggt ctg gtg gtg ggc ggg gct tcc acc tat gat gga ctg gcc 480
   Gly Gly Gly Leu Val Val Gly Gly Ala Ser Thr Tyr Asp Gly Leu Ala
   145 150 155 160
   ctc gct gcg cat gaa aac gtg gtg gtg gtg gcc atc cag tac cgc ctg 528
   Leu Ala Ala His Glu Asn Val Val Val Val Ala Ile Gln Tyr Arg Leu
   165 170 175
   ggc atc tgg gga ttc ttc agc aca ggg gac gaa cac agc cgg ggc aac      576
   Gly Ile Trp Gly Phe Phe Ser Thr Gly Asp Glu His Ser Arg Gly Asn
               180                 185                 190
   tgg ggt cac ttg gac cag gtg gcc gca ctg cac tgg gtc cag gag aac      624
   Trp Gly His Leu Asp Gln Val Ala Ala Leu His Trp Val Gln Glu Asn
           195                 200                 205
   atc gcc aac ttt gga ggc gac cca ggc tct gtg acc atc ttt gga gag      672
   Ile Ala Asn Phe Gly Gly Asp Pro Gly Ser Val Thr Ile Phe Gly Glu
       210                 215                 220
   tca gca gga ggg gaa agt gtc tct gtt ctg gtg ttg tct ccc ttg gcc      720
   Ser Ala Gly Gly Glu Ser Val Ser Val Leu Val Leu Ser Pro Leu Ala
   225                 230                 235                 240
   aag aac ctc ttc cac cgg gcc atc tct gag agt ggc gtg gcc ttc act      768
   Lys Asn Leu Phe His Arg Ala Ile Ser Glu Ser Gly Val Ala Phe Thr
                   245                 250                 255
   gct ggc ctg gtc agg aag gac atg aag gct gca gct aag caa att gct      816
   Ala Gly Leu Val Arg Lys Asp Met Lys Ala Ala Ala Lys Gln Ile Ala
               260                 265                 270
   gtc ctt gct ggg tgt aaa acc acc acc tcg gct gtc ttt gtt cac tgc      864
   Val Leu Ala Gly Cys Lys Thr Thr Thr Ser Ala Val Phe Val His Cys
           275                 280                 285
   ctg cgc cag aag tcg gag gac gag ctc ttg gac tta acg ctg aag atg      912
   Leu Arg Gln Lys Ser Glu Asp Glu Leu Leu Asp Leu Thr Leu Lys Met
       290                 295                 300
   aaa cct tta act ctt gat ttg cat gga gac ccc aga gag agc cat ccc      960
   Lys Pro Leu Thr Leu Asp Leu His Gly Asp Pro Arg Glu Ser His Pro
   305                 310                 315                 320
   ttc ctg acc act gtg gtg gat gga gtg ctg ctg ccc aag atg cct gaa     1008
   Phe Leu Thr Thr Val Val Asp Gly Val Leu Leu Pro Lys Met Pro Glu
                   325                 330                 335
   gag att ctg gct gaa aag gat ttc aac act gtc ccc tac atc gtg gga     1056
   Glu Ile Leu Ala Glu Lys Asp Phe Asn Thr Val Pro Tyr Ile Val Gly
               340                 345                 350
   atc aac aag caa gag ttt ggc tgg ctt ctg cca acg atg atg ggc ttc     1104
   Ile Asn Lys Gln Glu Phe Gly Trp Leu Leu Pro Thr Met Met Gly Phe
           355                 360                 365
   ccc ctc tct gaa ggc aag ctg gac cag aag acg gcc acg tca ctc ctg     1152
   Pro Leu Ser Glu Gly Lys Leu Asp Gln Lys Thr Ala Thr Ser Leu Leu
       370                 375                 380
   tgg aag tcc tac ccc atc gct aac atc cct gag gaa ctg act cca gtg     1200
   Trp Lys Ser Tyr Pro Ile Ala Asn Ile Pro Glu Glu Leu Thr Pro Val
   385                 390                 395                 400
   gcc act gac aag tat ttg ggg ggg aca gac gac ccc gtc aaa aag aaa     1248
   Ala Thr Asp Lys Tyr Leu Gly Gly Thr Asp Asp Pro Val Lys Lys Lys
                   405                 410                 415
   gac ctg ttc ctg gac ttg atg ggg gat gtg gtg ttt ggt gtc cca tct     1296
   Asp Leu Phe Leu Asp Leu Met Gly Asp Val Val Phe Gly Val Pro Ser
               420                 425                 430
   gtg acg gtg gcc cgt caa cac aga gat gca gga gcc ccc acc tac atg     1344
   Val Thr Val Ala Arg Gln His Arg Asp Ala Gly Ala Pro Thr Tyr Met
           435                 440                 445
   tat gag ttt cag tat cgc cca agc ttc tca tcg gac aag aaa ccc aag     1392
   Tyr Glu Phe Gln Tyr Arg Pro Ser Phe Ser Ser Asp Lys Lys Pro Lys
       450                 455                 460
   acg gtg atc ggg gac cac ggg gat gag atc ttc tcc gtc ttt ggg gct      1440
   Thr Val Ile Gly Asp His Gly Asp Glu Ile Phe Ser Val Phe Gly Ala
   465                 470                 475                 480
   cca ttt tta aga ggc gat gcc cca gaa gag gag gtc agt ctc agc aag      1488
   Pro Phe Leu Arg Gly Asp Ala Pro Glu Glu Glu Val Ser Leu Ser Lys
                   485                 490                 495
   acg gtg atg aaa ttc tgg gcc aac ttt gct cgc agt ggg aac ccc aat      1536
   Thr Val Met Lys Phe Trp Ala Asn Phe Ala Arg Ser Gly Asn Pro Asn
               500                 505                 510
   ggg gag ggg ctg ccc cat tgg ccg atg tac gac cag gaa gaa ggg tac      1584
   Gly Glu Gly Leu Pro His Trp Pro Met Tyr Asp Gln Glu Glu Gly Tyr
           515                 520                 525
   ctt cag atc ggc gtc aac acc cag gca gcc aag agg ctg aaa ggt gaa      1632
   Leu Gln Ile Gly Val Asn Thr Gln Ala Ala Lys Arg Leu Lys Gly Glu
       530                 535                 540
   gaa gtg gcc ttc tgg aac gat ctc ctg tcc aag gag gca gca aag aag      1680
   Glu Val Ala Phe Trp Asn Asp Leu Leu Ser Lys Glu Ala Ala Lys Lys
   545                 550                 555                 560
   cca ccc aag ata aag tga                                              1698
   Pro Pro Lys Ile Lys
                   565
<210> 6
   <211> 565
   <212> PRT
   <213> Artificial
<220>
   <223> Synthetic Construct
<400> 6
   Met Gly Ser Ser His His His His His His Ser Ser Gly Leu Val Pro
   1               5                   10                  15
   Arg Gly Ser His Met Gly Gln Pro Ala Ser Pro Pro Val Val Asp Thr
               20                  25                  30
   Ala Gln Gly Arg Val Leu Gly Lys Tyr Val Ser Leu Glu Gly Leu Ala
           35                  40                  45
   Gln Pro Val Ala Val Phe Leu Gly Val Pro Phe Ala Lys Pro Pro Leu
       50                  55                  60
   Gly Ser Leu Arg Phe Ala Pro Pro Gln Pro Ala Glu Pro Trp Ser Phe
   65                  70                  75                  80
   Val Lys Asn Thr Thr Ser Tyr Pro Pro Met Cys Cys Gln Asp Pro Val
                   85                  90                  95
   Ala Gly Gln Met Thr Ser Asp Leu Phe Thr Asn Arg Lys Glu Arg Leu
               100                 105                 110
   Ile Pro Glu Phe Ser Glu Asp Cys Leu Tyr Leu Asn Ile Tyr Thr Pro
           115                 120                 125
   Ala Asp Leu Thr Lys Arg Gly Arg Leu Pro Val Met Val Trp Ile His
       130                 135                 140
   Gly Gly Gly Leu Val Val Gly Gly Ala Ser Thr Tyr Asp Gly Leu Ala
   145                 150                 155                 160
   Leu Ala Ala His Glu Asn Val Val Val Val Ala Ile Gln Tyr Arg Leu
                   165                 170                 175
   Gly Ile Trp Gly Phe Phe Ser Thr Gly Asp Glu His Ser Arg Gly Asn
               180                 185                 190
   Trp Gly His Leu Asp Gln Val Ala Ala Leu His Trp Val Gln Glu Asn
           195                 200                 205
   Ile Ala Asn Phe Gly Gly Asp Pro Gly Ser Val Thr Ile Phe Gly Glu
       210                 215                 220
   Ser Ala Gly Gly Glu Ser Val Ser Val Leu Val Leu Ser Pro Leu Ala
   225                 230                 235                 240
   Lys Asn Leu Phe His Arg Ala Ile Ser Glu Ser Gly Val Ala Phe Thr
                   245                 250                 255
   Ala Gly Leu Val Arg Lys Asp Met Lys Ala Ala Ala Lys Gln Ile Ala
               260                 265                 270
   Val Leu Ala Gly Cys Lys Thr Thr Thr Ser Ala Val Phe Val His Cys
           275                 280                 285
   Leu Arg Gln Lys Ser Glu Asp Glu Leu Leu Asp Leu Thr Leu Lys Met
       290                 295                 300
   Lys Pro Leu Thr Leu Asp Leu His Gly Asp Pro Arg Glu Ser His Pro
   305                 310                 315                 320
   Phe Leu Thr Thr Val Val Asp Gly Val Leu Leu Pro Lys Met Pro Glu
                   325                 330                 335
   Glu Ile Leu Ala Glu Lys Asp Phe Asn Thr Val Pro Tyr Ile Val Gly
               340                 345                 350
   Ile Asn Lys Gln Glu Phe Gly Trp Leu Leu Pro Thr Met Met Gly Phe
           355                 360                 365
   Pro Leu Ser Glu Gly Lys Leu Asp Gln Lys Thr Ala Thr Ser Leu Leu
       370                 375                 380
   Trp Lys Ser Tyr Pro Ile Ala Asn Ile Pro Glu Glu Leu Thr Pro Val
   385                 390                 395                 400
   Ala Thr Asp Lys Tyr Leu Gly Gly Thr Asp Asp Pro Val Lys Lys Lys
                   405                 410                 415
   Asp Leu Phe Leu Asp Leu Met Gly Asp Val Val Phe Gly Val Pro Ser
               420                 425                 430
   Val Thr Val Ala Arg Gln His Arg Asp Ala Gly Ala Pro Thr Tyr Met
           435                 440                 445
   Tyr Glu Phe Gln Tyr Arg Pro Ser Phe Ser Ser Asp Lys Lys Pro Lys
       450                 455                 460
   Thr Val Ile Gly Asp His Gly Asp Glu Ile Phe Ser Val Phe Gly Ala
   465                 470                 475                 480
   Pro Phe Leu Arg Gly Asp Ala Pro Glu Glu Glu Val Ser Leu Ser Lys
                   485                 490                 495
   Thr Val Met Lys Phe Trp Ala Asn Phe Ala Arg Ser Gly Asn Pro Asn
               500                 505                 510
   Gly Glu Gly Leu Pro His Trp Pro Met Tyr Asp Gln Glu Glu Gly Tyr
           515                 520                 525
   Leu Gln Ile Gly Val Asn Thr Gln Ala Ala Lys Arg Leu Lys Gly Glu
       530                 535                 540
   Glu Val Ala Phe Trp Asn Asp Leu Leu Ser Lys Glu Ala Ala Lys Lys
   545                 550                 555                 560
   Pro Pro Lys Ile Lys
                   565
<210> 7
   <211> 1698
   <212> DNA
   <213> Artificial
<220>
   <223> Mutante der PLE
<220>
   <221> CDS
   <222> (1)..(1698)
<400> 7
   atg ggc agc agc cat cat cat cat cat cac agc agc ggc ctg gtg ccg       48
   Met Gly Ser Ser His His His His His His Ser Ser Gly Leu Val Pro
   1               5                   10                  15
   cgc ggc agc cat atg ggg cag cca gcc tcg ccg cct gtt gtg gac act       96
   Arg Gly Ser His Met Gly Gln Pro Ala Ser Pro Pro Val Val Asp Thr
               20                  25                  30
   gcc cag ggc cga gtc ctg ggg aag tac gtc agc tta gaa ggc ctg gca      144
   Ala Gln Gly Arg Val Leu Gly Lys Tyr Val Ser Leu Glu Gly Leu Ala
           35                  40                  45
   cag ccg gtg gcc gtc ttc ctg gga gtc cct ttt gcc aag ccc cct ctc      192
   Gln Pro Val Ala Val Phe Leu Gly Val Pro Phe Ala Lys Pro Pro Leu
       50                  55                  60
   gga tcc ttg agg ttt gct ccg ccg cag cct gca gaa cca tgg agc ttc      240
   Gly Ser Leu Arg Phe Ala Pro Pro Gln Pro Ala Glu Pro Trp Ser Phe
   65                  70                  75                  80
   gtg aag aac acc acc tcc tac cct ccc atg tgc tgc cag gac cca gta      288
   Val Lys Asn Thr Thr Ser Tyr Pro Pro Met Cys Cys Gln Asp Pro Val
                   85                  90                  95
   gcg ggg cag atg acc tca gat cta ttt acc aac aga aag gag agg ctc      336
   Ala Gly Gln Met Thr Ser Asp Leu Phe Thr Asn Arg Lys Glu Arg Leu
               100                 105                 110
   att ccg gag ttt tct gaa gac tgt ctc tac cta aat att tac acc cct      384
   Ile Pro Glu Phe Ser Glu Asp Cys Leu Tyr Leu Asn Ile Tyr Thr Pro
           115                 120                 125
   gct gac ctg aca aag agg ggc aga ctg ccg gtg atg gtg tgg atc cac      432
   Ala Asp Leu Thr Lys Arg Gly Arg Leu Pro Val Met Val Trp Ile His
       130                 135                 140
   gga gga ggt ctg gtg gtg ggc ggg gct tcc acc tat gat gga ctg gcc      480
   Gly Gly Gly Leu Val Val Gly Gly Ala Ser Thr Tyr Asp Gly Leu Ala
   145                 150                 155                 160
   ctc gct gcg cat gaa aac gtg gtg gtg gtg gcc atc cag tac cgc ctg      528
   Leu Ala Ala His Glu Asn Val Val Val Val Ala Ile Gln Tyr Arg Leu
                   165                 170                 175
   ggc atc tgg gga ttc ttc agc aca ggg gac gaa cac agc cgg ggc aac      576
   Gly Ile Trp Gly Phe Phe Ser Thr Gly Asp Glu His Ser Arg Gly Asn
               180                 185                 190
   tgg ggt cac ttg gac cag gtg gcc gca ctg cac tgg gtc cag gag aac      624
   Trp Gly His Leu Asp Gln Val Ala Ala Leu His Trp Val Gln Glu Asn
           195                 200                 205
   atc gcc aac ttt gga ggc gac cca ggc tct gtg acc atc ttt gga gag      672
   Ile Ala Asn Phe Gly Gly Asp Pro Gly Ser Val Thr Ile Phe Gly Glu
       210                 215                 220
   tca gca gga ggg gaa agt gtc tct gtt ctg gtg ttg tct ccc ttg gcc      720
   Ser Ala Gly Gly Glu Ser Val Ser Val Leu Val Leu Ser Pro Leu Ala
   225                 230                 235                 240
   aag aac ctc ttc cac cgg gcc atc tct gag agt ggc gtg gcc ttc act      768
   Lys Asn Leu Phe His Arg Ala Ile Ser Glu Ser Gly Val Ala Phe Thr
                   245                 250                 255
   gct ggc ctg gtc agg aag gac atg aag gct gca gct aag caa att gct      816
   Ala Gly Leu Val Arg Lys Asp Met Lys Ala Ala Ala Lys Gln Ile Ala
               260                 265                 270
   gtc ctt gct ggg tgt aaa acc acc acc tcg gct gtc ttt gtt cac tgc      864
   Val Leu Ala Gly Cys Lys Thr Thr Thr Ser Ala Val Phe Val His Cys
           275                 280                 285
   ctg cgc cag aag tcg gag gac gag ctc ttg gac tta acg ctg aag atg      912
   Leu Arg Gln Lys Ser Glu Asp Glu Leu Leu Asp Leu Thr Leu Lys Met
       290                 295                 300
   aaa ttt ttc gct ctt gat ttg cat gga gac ccc aga gag agc cat ccc      960
   Lys Phe Phe Ala Leu Asp Leu His Gly Asp Pro Arg Glu Ser His Pro
   305                 310                 315                 320
   ttc ctg acc act gtg gtg gat gga gtg ctg ctg ccc aag atg cct gaa     1008
   Phe Leu Thr Thr Val Val Asp Gly Val Leu Leu Pro Lys Met Pro Glu
                   325                 330                 335
   gag att ctg gct gaa aag gat ttc aac act gtc ccc tac atc gtg gga     1056
   Glu Ile Leu Ala Glu Lys Asp Phe Asn Thr Val Pro Tyr Ile Val Gly
               340                 345                 350
   atc aac aag caa gag ttt ggc tgg ctt ctg cca acg atg atg ggc ttc     1104
   Ile Asn Lys Gln Glu Phe Gly Trp Leu Leu Pro Thr Met Met Gly Phe
           355                 360                 365
   ccc ctc tct gaa ggc aag ctg gac cag aag acg gcc acg tca ctc ctg     1152
   Pro Leu Ser Glu Gly Lys Leu Asp Gln Lys Thr Ala Thr Ser Leu Leu
       370                 375                 380
   tgg aag tcc tac ccc atc gct aac atc cct gag gaa ctg act cca gtg     1200
   Trp Lys Ser Tyr Pro Ile Ala Asn Ile Pro Glu Glu Leu Thr Pro Val
   385                 390                 395                 400
   gcc act gac aag tat ttg ggg ggg aca gac gac ccc gtc aaa aag aaa     1248
   Ala Thr Asp Lys Tyr Leu Gly Gly Thr Asp Asp Pro Val Lys Lys Lys
                   405                 410                 415
   gac ctg ttc ctg gac ttg atg ggg gat gtg gtg ttt ggt gtc cca tct     1296
   Asp Leu Phe Leu Asp Leu Met Gly Asp Val Val Phe Gly Val Pro Ser
               420                 425                 430
   gtg acg gtg gcc cgt caa cac aga gat gca gga gcc ccc acc tac atg     1344
   Val Thr Val Ala Arg Gln His Arg Asp Ala Gly Ala Pro Thr Tyr Met
           435                 440                 445
   tat gag ttt cag tat cgc cca agc ttc tca tcg gac aag aaa ccc aag     1392
   Tyr Glu Phe Gln Tyr Arg Pro Ser Phe Ser Ser Asp Lys Lys Pro Lys
       450                 455                 460
   acg gtg atc ggg gac cac ggg gat gag atc ttc tcc gtc ttt ggg gct     1440
   Thr Val Ile Gly Asp His Gly Asp Glu Ile Phe Ser Val Phe Gly Ala
   465                 470                 475                 480
   cca ttt tta aga ggc gat gcc cca gaa gag gag gtc agt ctc agc aag     1488
   Pro Phe Leu Arg Gly Asp Ala Pro Glu Glu Glu Val Ser Leu Ser Lys
                   485                 490                 495
   acg gtg atg aaa ttc tgg gcc aac ttt gct cgc agt ggg aac ccc aat     1536
   Thr Val Met Lys Phe Trp Ala Asn Phe Ala Arg Ser Gly Asn Pro Asn
               500                 505                 510
   ggg gag ggg ctg ccc cat tgg ccg atg tac gac cag gaa gaa ggg tac     1584
   Gly Glu Gly Leu Pro His Trp Pro Met Tyr Asp Gln Glu Glu Gly Tyr
           515                 520                 525
   ctt cag atc ggc gtc aac acc cag gca gcc aag agg ctg aaa ggt gaa     1632
   Leu Gln Ile Gly Val Asn Thr Gln Ala Ala Lys Arg Leu Lys Gly Glu
       530                 535                 540
   gaa gtg gcc ttc tgg aac gat ctc ctg tcc aag gag gca gca aag aag     1680
   Glu Val Ala Phe Trp Asn Asp Leu Leu Ser Lys Glu Ala Ala Lys Lys
   545                 550                 555                 560
   cca ccc aag ata aag tga                                             1698
   Pro Pro Lys Ile Lys
                   565
<210> 8
   <211> 565
   <212> PRT
   <213> Artificial
<220>
   <223> Synthetic Construct
<400> 8
   Met Gly Ser Ser His His His His His His Ser Ser Gly Leu Val Pro
   1               5                   10                  15
   Arg Gly Ser His Met Gly Gln Pro Ala Ser Pro Pro Val Val Asp Thr
               20                  25                  30
   Ala Gln Gly Arg Val Leu Gly Lys Tyr Val Ser Leu Glu Gly Leu Ala
           35                  40                  45
   Gln Pro Val Ala Val Phe Leu Gly Val Pro Phe Ala Lys Pro Pro Leu
       50                  55                  60
   Gly Ser Leu Arg Phe Ala Pro Pro Gln Pro Ala Glu Pro Trp Ser Phe
   65                  70                  75                  80
   Val Lys Asn Thr Thr Ser Tyr Pro Pro Met Cys Cys Gln Asp Pro Val
                   85                  90                  95
   Ala Gly Gln Met Thr Ser Asp Leu Phe Thr Asn Arg Lys Glu Arg Leu
               100                 105                 110
   Ile Pro Glu Phe Ser Glu Asp Cys Leu Tyr Leu Asn Ile Tyr Thr Pro
           115                 120                 125
   Ala Asp Leu Thr Lys Arg Gly Arg Leu Pro Val Met Val Trp Ile His
       130                 135                 140
   Gly Gly Gly Leu Val Val Gly Gly Ala Ser Thr Tyr Asp Gly Leu Ala
   145                 150                 155                 160
   Leu Ala Ala His Glu Asn Val Val Val Val Ala Ile Gln Tyr Arg Leu
                   165                 170                 175
   Gly Ile Trp Gly Phe Phe Ser Thr Gly Asp Glu His Ser Arg Gly Asn
               180                 185                 190
   Trp Gly His Leu Asp Gln Val Ala Ala Leu His Trp Val Gln Glu Asn
           195                 200                 205
   Ile Ala Asn Phe Gly Gly Asp Pro Gly Ser Val Thr Ile Phe Gly Glu
       210                 215                 220
   Ser Ala Gly Gly Glu Ser Val Ser Val Leu Val Leu Ser Pro Leu Ala
   225                 230                 235                 240
   Lys Asn Leu Phe His Arg Ala Ile Ser Glu Ser Gly Val Ala Phe Thr
                   245                 250                 255
   Ala Gly Leu Val Arg Lys Asp Met Lys Ala Ala Ala Lys Gln Ile Ala
               260                 265                 270
   Val Leu Ala Gly Cys Lys Thr Thr Thr Ser Ala Val Phe Val His Cys
           275                 280                 285
   Leu Arg Gln Lys Ser Glu Asp Glu Leu Leu Asp Leu Thr Leu Lys Met
       290                 295                 300
   Lys Phe Phe Ala Leu Asp Leu His Gly Asp Pro Arg Glu Ser His Pro
   305                 310                 315                 320
   Phe Leu Thr Thr Val Val Asp Gly Val Leu Leu Pro Lys Met Pro Glu
                   325                 330                 335
   Glu Ile Leu Ala Glu Lys Asp Phe Asn Thr Val Pro Tyr Ile Val Gly
               340                 345                 350
   Ile Asn Lys Gln Glu Phe Gly Trp Leu Leu Pro Thr Met Met Gly Phe
           355                 360                 365
   Pro Leu Ser Glu Gly Lys Leu Asp Gln Lys Thr Ala Thr Ser Leu Leu
       370                 375                 380
   Trp Lys Ser Tyr Pro Ile Ala Asn Ile Pro Glu Glu Leu Thr Pro Val
   385                 390                 395                 400
   Ala Thr Asp Lys Tyr Leu Gly Gly Thr Asp Asp Pro Val Lys Lys Lys
                   405                 410                 415
   Asp Leu Phe Leu Asp Leu Met Gly Asp Val Val Phe Gly Val Pro Ser
               420                 425                 430
   Val Thr Val Ala Arg Gln His Arg Asp Ala Gly Ala Pro Thr Tyr Met
           435                 440                 445
   Tyr Glu Phe Gln Tyr Arg Pro Ser Phe Ser Ser Asp Lys Lys Pro Lys
       450                 455                 460
   Thr Val Ile Gly Asp His Gly Asp Glu Ile Phe Ser Val Phe Gly Ala
   465                 470                 475                 480
   Pro Phe Leu Arg Gly Asp Ala Pro Glu Glu Glu Val Ser Leu Ser Lys
                   485                 490                 495
   Thr Val Met Lys Phe Trp Ala Asn Phe Ala Arg Ser Gly Asn Pro Asn
               500                 505                 510
   Gly Glu Gly Leu Pro His Trp Pro Met Tyr Asp Gln Glu Glu Gly Tyr
           515                 520                 525
   Leu Gln Ile Gly Val Asn Thr Gln Ala Ala Lys Arg Leu Lys Gly Glu
       530                 535                 540
   Glu Val Ala Phe Trp Asn Asp Leu Leu Ser Lys Glu Ala Ala Lys Lys
   545                 550                 555                 560
   Pro Pro Lys Ile Lys
                   565
<210> 9
   <211> 1698
   <212> DNA
   <213> Artificial
<220>
   <223> Mutante der PLE
<220>
   <221> CDS
   <222> (1)..(1698)
<400> 9
   atg ggc agc agc cat cat cat cat cat cac agc agc ggc ctg gtg ccg      48
   Met Gly Ser Ser His His His His His His Ser Ser Gly Leu Val Pro
   1               5                   10                  15
   cgc ggc agc cat atg ggg cag cca gcc tcg ccg cct gtt gtg gac act      96
   Arg Gly Ser His Met Gly Gln Pro Ala Ser Pro Pro Val Val Asp Thr
               20                  25                  30
   gcc cag ggc cga gtc ctg ggg aag tac gtc agc tta gaa ggc ctg gca     144
   Ala Gln Gly Arg Val Leu Gly Lys Tyr Val Ser Leu Glu Gly Leu Ala
           35                  40                  45
   cag ccg gtg gcc gtc ttc ctg gga gtc cct ttt gcc aag ccc cct ctc     192
   Gln Pro Val Ala Val Phe Leu Gly Val Pro Phe Ala Lys Pro Pro Leu
       50                  55                  60
   gga tcc ttg agg ttt gct ccg ccg cag cct gca gaa cca tgg agc ttc     240
   Gly Ser Leu Arg Phe Ala Pro Pro Gln Pro Ala Glu Pro Trp Ser Phe
   65                  70                  75                  80
   gtg aag aac acc acc tcc tac cct ccc atg tgc tgc caa gag cca att     288
   Val Lys Asn Thr Thr Ser Tyr Pro Pro Met Cys Cys Gln Glu Pro Ile
                   85                  90                  95
   ggg gga cag atg ctc tca gat cta ttt acc aac aga aag gag agg ctc     336
   Gly Gly Gln Met Leu Ser Asp Leu Phe Thr Asn Arg Lys Glu Arg Leu
               100                 105                 110
   att ccg gag ttt tct gaa gac tgt ctc tac cta aat att tac acc cct     384
   Ile Pro Glu Phe Ser Glu Asp Cys Leu Tyr Leu Asn Ile Tyr Thr Pro
           115                 120                 125
   gct gac ctg aca aag agg ggc aga ctg ccg gtg atg gtg tgg atc cac     432
   Ala Asp Leu Thr Lys Arg Gly Arg Leu Pro Val Met Val Trp Ile His
       130                 135                 140
   gga gga ggt ctg gtg gtg ggc ggg gct tcc acc tat gat gga ctg gcc     480
   Gly Gly Gly Leu Val Val Gly Gly Ala Ser Thr Tyr Asp Gly Leu Ala
   145                 150                 155                 160
   ctc gct gcg cat gaa aac gtg gtg gtg gtg gcc atc cag tac cgc ctg      528
   Leu Ala Ala His Glu Asn Val Val Val Val Ala Ile Gln Tyr Arg Leu
                   165                 170                 175
   ggc atc tgg gga ttc ttc agc aca ggg gac gaa cac agc cgg ggc aac      576
   Gly Ile Trp Gly Phe Phe Ser Thr Gly Asp Glu His Ser Arg Gly Asn
               180                 185                 190
   tgg ggt cac ttg gac cag gtg gcc gca ctg cac tgg gtc cag gag aac      624
   Trp Gly His Leu Asp Gln Val Ala Ala Leu His Trp Val Gln Glu Asn
           195                 200                 205
   atc gcc aac ttt gga ggc gac cca ggc tct gtg acc atc ttt gga gag      672
   Ile Ala Asn Phe Gly Gly Asp Pro Gly Ser Val Thr Ile Phe Gly Glu
       210                 215                 220
   tca gca gga ggg gaa agt gtc tct gtt ctg gtg ttg tct ccc ttg gcc      720
   Ser Ala Gly Gly Glu Ser Val Ser Val Leu Val Leu Ser Pro Leu Ala
   225                 230                 235                 240
   aag aac ctc ttc cac cgg gcc atc tct gag agt ggc gtg gcc ttc act      768
   Lys Asn Leu Phe His Arg Ala Ile Ser Glu Ser Gly Val Ala Phe Thr
                   245                 250                 255
   gct ggc ctg gtc agg aag gac atg aag gct gca gct aag caa att gct      816
   Ala Gly Leu Val Arg Lys Asp Met Lys Ala Ala Ala Lys Gln Ile Ala
               260                 265                 270
   gtc ctt gct ggg tgt aaa acc acc acc tcg gct gtc ttt gtt cac tgc      864
   Val Leu Ala Gly Cys Lys Thr Thr Thr Ser Ala Val Phe Val His Cys
           275                 280                 285
   ctg cgc cag aag tcg gag gac gag ctc ttg gac tta acg ctg aag atg      912
   Leu Arg Gln Lys Ser Glu Asp Glu Leu Leu Asp Leu Thr Leu Lys Met
       290                 295                 300
   aaa cct tta act ctt gat ttg cat gga gac ccc aga gag agc cat ccc      960
   Lys Pro Leu Thr Leu Asp Leu His Gly Asp Pro Arg Glu Ser His Pro
   305                 310                 315                 320
   ttc ctg acc act gtg gtg gat gga gtg ctg ctg ccc aag atg cct gaa      1008
   Phe Leu Thr Thr Val Val Asp Gly Val Leu Leu Pro Lys Met Pro Glu
                   325                 330                 335
   gag att ctg gct gaa aag gat ttc aac act gtc ccc tac atc gtg gga      1056
   Glu Ile Leu Ala Glu Lys Asp Phe Asn Thr Val Pro Tyr Ile Val Gly
               340                 345                 350
   atc aac aag caa gag ttt ggc tgg ctt ctg cca acg atg atg ggc ttc      1104
   Ile Asn Lys Gln Glu Phe Gly Trp Leu Leu Pro Thr Met Met Gly Phe
           355                 360                 365
   ccc ctc tct gaa ggc aag ctg gac cag aag acg gcc acg tca ctc ctg      1152
   Pro Leu Ser Glu Gly Lys Leu Asp Gln Lys Thr Ala Thr Ser Leu Leu
       370                 375                 380
   tgg aag tcc tac ccc atc gct aac atc cct gag gaa ctg act cca gtg      1200
   Trp Lys Ser Tyr Pro Ile Ala Asn Ile Pro Glu Glu Leu Thr Pro Val
   385                 390                 395                 400
   gcc act gac aag tat ttg ggg ggg aca gac gac ccc gtc aaa aag aaa      1248
   Ala Thr Asp Lys Tyr Leu Gly Gly Thr Asp Asp Pro Val Lys Lys Lys
                   405                 410                 415
   gac ctg ttc ctg gac ttg atg ggg gat gtg gtg ttt ggt gtc cca tct      1296
   Asp Leu Phe Leu Asp Leu Met Gly Asp Val Val Phe Gly Val Pro Ser
               420                 425                 430
   gtg acg gtg gcc cgt caa cac aga gat gca gga gcc ccc acc tac atg      1344
   Val Thr Val Ala Arg Gln His Arg Asp Ala Gly Ala Pro Thr Tyr Met
           435                 440                 445
   tat gag ttt cag tat cgc cca agc ttc tca tcg gac aag aaa ccc aag      1392
   Tyr Glu Phe Gln Tyr Arg Pro Ser Phe Ser Ser Asp Lys Lys Pro Lys
       450                 455                 460
   acg gtg atc ggg gac cac ggg gat gag atc ttc tcc gtc ttt ggt ttt      1440
   Thr Val Ile Gly Asp His Gly Asp Glu Ile Phe Ser Val Phe Gly Phe
   465                 470                 475                 480
   cca ctg tta aaa ggc gat gcc cca gaa gag gag gtc agt ctc agc aag      1488
   Pro Leu Leu Lys Gly Asp Ala Pro Glu Glu Glu Val Ser Leu Ser Lys
                   485                 490                 495
   acg gtg atg aaa ttc tgg gcc aac ttt gct cgc agt ggg aac ccc aat      1536
   Thr Val Met Lys Phe Trp Ala Asn Phe Ala Arg Ser Gly Asn Pro Asn
               500                 505                 510
   ggg gag ggg ctg ccc cat tgg ccg atg tac gac cag gaa gaa ggg tac      1584
   Gly Glu Gly Leu Pro His Trp Pro Met Tyr Asp Gln Glu Glu Gly Tyr
           515                 520                 525
   ctt cag atc ggc gtc aac acc cag gca gcc aag agg ctg aaa ggt gaa      1632
   Leu Gln Ile Gly Val Asn Thr Gln Ala Ala Lys Arg Leu Lys Gly Glu
       530                 535                 540
   gaa gtg gcc ttc tgg aac gat ctc ctg tcc aag gag gca gca aag aag      1680
   Glu Val Ala Phe Trp Asn Asp Leu Leu Ser Lys Glu Ala Ala Lys Lys
   545                 550                 555                 560
   cca ccc aag ata aag tga                                              1698
   Pro Pro Lys Ile Lys
                   565
<210> 10
   <211> 565
   <212> PRT
   <213> Artificial
<220>
   <223> Synthetic Construct
<400> 10
   Met Gly Ser Ser His His His His His His Ser Ser Gly Leu Val Pro
   1               5                   10                  15
   Arg Gly Ser His Met Gly Gln Pro Ala Ser Pro Pro Val Val Asp Thr
               20                  25                  30
   Ala Gln Gly Arg Val Leu Gly Lys Tyr Val Ser Leu Glu Gly Leu Ala
           35                  40                  45
   Gln Pro Val Ala Val Phe Leu Gly Val Pro Phe Ala Lys Pro Pro Leu
       50                  55                  60
   Gly Ser Leu Arg Phe Ala Pro Pro Gln Pro Ala Glu Pro Trp Ser Phe
   65                  70                  75                  80
   Val Lys Asn Thr Thr Ser Tyr Pro Pro Met Cys Cys Gln Glu Pro Ile
                   85                  90                  95
   Gly Gly Gln Met Leu Ser Asp Leu Phe Thr Asn Arg Lys Glu Arg Leu
               100                 105                 110
   Ile Pro Glu Phe Ser Glu Asp Cys Leu Tyr Leu Asn Ile Tyr Thr Pro
           115                 120                 125
   Ala Asp Leu Thr Lys Arg Gly Arg Leu Pro Val Met Val Trp Ile His
       130                 135                 140
   Gly Gly Gly Leu Val Val Gly Gly Ala Ser Thr Tyr Asp Gly Leu Ala
   145                 150                 155                 160
   Leu Ala Ala His Glu Asn Val Val Val Val Ala Ile Gln Tyr Arg Leu
                   165                 170                 175
   Gly Ile Trp Gly Phe Phe Ser Thr Gly Asp Glu His Ser Arg Gly Asn
               180                 185                 190
   Trp Gly His Leu Asp Gln Val Ala Ala Leu His Trp Val Gln Glu Asn
           195                 200                 205
   Ile Ala Asn Phe Gly Gly Asp Pro Gly Ser Val Thr Ile Phe Gly Glu
       210                 215                 220
   Ser Ala Gly Gly Glu Ser Val Ser Val Leu Val Leu Ser Pro Leu Ala
   225                 230                 235                 240
   Lys Asn Leu Phe His Arg Ala Ile Ser Glu Ser Gly Val Ala Phe Thr
                   245                 250                 255
   Ala Gly Leu Val Arg Lys Asp Met Lys Ala Ala Ala Lys Gln Ile Ala
               260                 265                 270
   Val Leu Ala Gly Cys Lys Thr Thr Thr Ser Ala Val Phe Val His Cys
           275                 280                 285
   Leu Arg Gln Lys Ser Glu Asp Glu Leu Leu Asp Leu Thr Leu Lys Met
       290                 295                 300
   Lys Pro Leu Thr Leu Asp Leu His Gly Asp Pro Arg Glu Ser His Pro
   305                 310                 315                 320
   Phe Leu Thr Thr Val Val Asp Gly Val Leu Leu Pro Lys Met Pro Glu
                   325                 330                 335
   Glu Ile Leu Ala Glu Lys Asp Phe Asn Thr Val Pro Tyr Ile Val Gly
               340                 345                 350
   Ile Asn Lys Gln Glu Phe Gly Trp Leu Leu Pro Thr Met Met Gly Phe
           355                 360                 365
   Pro Leu Ser Glu Gly Lys Leu Asp Gln Lys Thr Ala Thr Ser Leu Leu
       370                 375                 380
   Trp Lys Ser Tyr Pro Ile Ala Asn Ile Pro Glu Glu Leu Thr Pro Val
   385                 390                 395                 400
   Ala Thr Asp Lys Tyr Leu Gly Gly Thr Asp Asp Pro Val Lys Lys Lys
                   405                 410                 415
   Asp Leu Phe Leu Asp Leu Met Gly Asp Val Val Phe Gly Val Pro Ser
               420                 425                 430
   Val Thr Val Ala Arg Gln His Arg Asp Ala Gly Ala Pro Thr Tyr Met
           435                 440                 445
   Tyr Glu Phe Gln Tyr Arg Pro Ser Phe Ser Ser Asp Lys Lys Pro Lys
       450                 455                 460
   Thr Val Ile Gly Asp His Gly Asp Glu Ile Phe Ser Val Phe Gly Phe
   465                 470                 475                 480
   Pro Leu Leu Lys Gly Asp Ala Pro Glu Glu Glu Val Ser Leu Ser Lys
                   485                 490                 495
   Thr Val Met Lys Phe Trp Ala Asn Phe Ala Arg Ser Gly Asn Pro Asn
               500                 505                 510
   Gly Glu Gly Leu Pro His Trp Pro Met Tyr Asp Gln Glu Glu Gly Tyr
           515                 520                 525
   Leu Gln Ile Gly Val Asn Thr Gln Ala Ala Lys Arg Leu Lys Gly Glu
       530                 535                 540
   Glu Val Ala Phe Trp Asn Asp Leu Leu Ser Lys Glu Ala Ala Lys Lys
   545                 550                 555                 560
   Pro Pro Lys Ile Lys
                   565
<210> 11
   <211> 26
   <212> DNA
   <213> Artificial
<220>
   <223> Primer
<400> 11
   cacccatatg gggcagccag cctcgc 26
<210> 12
   <211> 36
   <212> DNA
   <213> Artificial
<220>
   <223> Primer
<400> 12
   ccgctcgagt cactttatct tgggtggctt ctttgc 36

## Beschreibung

Die vorliegende Erfindung bezieht sich auf die in den Ansprüchen aufgeführten Isoformen der Schweineleber Esterase (γPLE), sie enthaltende Vehikel und deren Anwendung in der Herstellung von enantiomerenangereicherten Alkoholen bzw. Estern.

Lipasen und Esterasen eignen sich als effiziente Biokatalysatoren zur Darstellung einer Vielzahl optisch aktiver Verbindungen. Während jedoch eine ganze Reihe von Lipasen - insbesondere mikrobiellen Ursprungs - kommerziell erhältlich sind, stehen nur sehr wenige Esterasen für den Einsatz in einer Racematspaltung in technischen Mengen zur Verfügung [Bornscheuer, U.T. und Kazlauskas R.J., Hydrolases in Organic Synthesis (2005), 2nd ed, Wiley-VCH, Weinheim].

Besonderes Interesse gilt dabei der Schweineleberesterase aufgrund ihrer interessanten katalytischen Eigenschaften in der organischen Synthese [Faber, K., Biotransformations in Organic Chemistry (2004), 5th ed. Springer, Berlin; Jones, J.B. Pure Appl. Chem, (1990), 62, 1445-1448, Jones et. al. Can. J. Chem. (1985), 63, 452-456; Lam, L.K.P. et. al., J. Org. Chem. (1986), 51, 2047-2050).

Obwohl gezeigt werden konnte, dass mit Esteraseextrakten aus Schweinelebergewebe teilweise Substrate mit guter Stereoselektivität umgesetzt werden können, ist die Verwendung solcher Extrakte allerdings mit einer Reihe von Nachteilen verbunden. Neben Schwankungen des Esteraseanteils zwischen verschiedenen Chargen ist insbesondere die Anwesenheit weiterer Hydrolasen als problematisch bezüglich der Stereoselektivitäten anzusehen (Seebach, D. et. al, 25 Chimia (1986), 40, 315-318). Zusätzlich besteht das Problem, dass die herkömmlichen Extrakte in Form mehrerer Isoenzyme vorliegen (Farb, D., et al, Arch. Biochem. Biophys. (1980) 203, 214-226), die sich teilweise in ihrer Substratspezifität erheblich unterscheiden. Heymann, E. und Junge, W. (Eur. J. Biochem. (1979), 95, 509-518; Eur. J. Biochem. (1979), 95, 519-525) gelang eine aufwendige elektrophoretische Trennung, so dass Fraktionen isoliert werden konnten, die bevorzugt Butyrylcholin, Prolin-β-naphthylamid und Methylbutyrat spalten. Im Gegensatz dazu zeigen andere Untersuchungen (z. B. Lam, L.K.P., et. al, J. Am. Chem. Soc. (1988) 110, 4409-4411) lediglich Unterschiede in der Aktivität, nicht aber in der Spezifität einzelner Fraktionen.

Die Klonierung putativer Schweineleberesterase-Gene ist zwar bereits seit längerem bekannt (Takahashi, T, et. al., J. Biol. Chem. (1989), 264, 11565-11571; FEBS Lett. (1991), 280, 297-300; FEBS Lett. (1991), 293, 37-41; David, L. et. al, Eur, J. Biochem. (1998) 257, 142-148), die funktionelle, rekombinante Expression einer aktiven Schweineleberesterase ist bisher nur in Pichia pastoris (Lange, S. et al., ChemBioChem (2001), 2, 576-582) bzw. E. coli (DE 10061864) beschrieben worden.

Ebenfalls in der Literatur beschrieben sind Zusätze zum Medium während der Expression in E. coli. Die Zugabe von Ethanol bis zu 3% (v/v) zum Medium induziert die Bildung von E. coli eigenen Chaperonen, Enzymen die als Faltungshilfen dienen und in der Regel die korrekte Faltung unterstützen (Thomas, JG, Protein Expression and Purif (1997), 11, 289-296). Die Expression der Schweineleberesterase in E. coli Origami unter Zugabe von 3% (v/v) Ethanol zum Medium führte jedoch zu keiner nachweisbaren aktiven Expression der Esterase in E. coli, sondern nur zu Inclusion Bodies.

In der DE10061864 wird die Coexpression von bestimmten Chaperonen und der γPLE vorgeschlagen. Dergestalt konnte erstmals aktive γPLE aus E. coli erzeugt werden.

Zusammenfassend kann gesagt werden, dass die Expression der nativen Schweineleberesterase aus E. coli zwar möglich, jedoch noch nicht im industriellen Maßstab etabliert ist. Es ist weiterhin sinnvoll und notwendig, die (Substrat-)Aktivitäten der Schweineleberesterasen zu verbessern, um darüber hinaus verbesserte Systeme zu erhalten, die bevorzugt im technischen Maßstab im Rahmen der biosynthetischen Herstellung von chemischen Zwischenprodukten eingesetzt werden können.

Aufgabe der vorliegenden Erfindung war daher die Angabe neuer, gegenüber dem Stand der Technik verbesserter Esterasen. Es sollten neue Esterasen mit verbesserter Aktivität und/oder Selektivität und/oder Stabilität bereitgestellt werden. Insbesondere im Hinblick auf Raum/ZeitAusbeute und Enantioselektivität bei der Umsetzung sowie geänderter oder erweiterter Substratspezifität sollten diese Esterasen denen des Standes der Technik überlegen sein.

Diese Aufgabe wird anspruchsgemäß gelöst.

Durch das Bereitstellen von Esterasen gemäß Seq. ID Nr. 2, aufweisend mindestens eine Mutation, ausgewählt aus der Gruppe bestehend aus:

| Position | Aminosäure |
|---|---|
| 94 | E |
| 96 | I |
| 97 | A, G |
| 98 | G |
| 101 | L |
| 108 | R |
| 113 | I |
| 114 | P |
| 150 | V |
| 154 | S |
| 155 | T |
| 159 | L |
| 160 | A |
| 255 | F |
| 257 | A |
| 258 | G |
| 306 | P |
| 307 | F |
| 308 | A |
| 311 | L |
| 315 | P |
| 323 | T |
| 480 | A |
| 482 | F |
| 484 | R, |

gelangt man überraschenderweise zu Spezies, die die genannten Aufgaben erfüllen. Insbesondere kann durch Mutationen an diesen Stellen die Substratspezifität die Enantioselektivität und/oder die Aktivität der nativen γPLE variiert und verbessert werden. Die Position bezieht sich dabei naturgemäß auf die erste Aminosäure der Seq. ID No 2.

Anspruchs gemäß sind Esterasen gemäß Seq. ID Nr. 4, 6, 8 und 10. Diese weisen gegenüber der native γPLE bessere Aktivität und/oder Selektivität und/oder Stabilität auf. Insbesondere im Hinblick auf die Aktivitäten, Enantioselektivitäten bzw. andere Substratspezifitäten zeichnen sich die erfindungsgemäßen Esterasen aus.

In einer weiteren Ausführungsform bezieht sich die vorliegende Erfindung auf isolierte Nukleinsäure codierend für eine erfindungsgemäße Esterase wie in den Ansprüchen definiert Nukleinsäuresequenzen sind solche der Seq. ID Nr. 3, 5, 7 und 9 oder deren komplementäre Form.

In einer weiteren Ausgestaltung bezieht sich die vorliegende Erfindung auf Gene, rekombinante Expressionssysteme (z.B. Mikroorganismen) oder rekombinante Plasmide/Vektoren aufweisend eine oder mehrere der erfindungsgemäßen Nukleinsäuren wie in den Ansprüchen definiert.

Unter einem Expressionssystem ist ein System zur rekombinanten Expression der erfindungsgemäßen Nukleinsäuren und damit zur rekombinanten Herstellung der erfindungsgemäßen Polypeptide zu verstehen. Diese Herstellung kann bevorzugt in mit entsprechenden Nukleinsäuresequenzen oder Vektoren (s.u.) transformierten oder transfizierten (die Begriffe "Transformation" und "Transfektion" werden gemäß dieser Erfindung sinngleich verwendet) Mikroorganismen oder anderen Wirten erfolgen. Die Transformation bzw. Transfektion kann nach bekannten Methoden erfolgen, z.B. durch Calciumphosphat-Copräzipitation, Lipofektion, Elektroporation, PEG/DMSO-Methode, Partikelbeschuß oder virale/bakteriophage Infektion. Die erfindungsgemäße Zelle kann die rekombinante Nukleinsäure in extrachromosomaler oder chromosomal integrierter Form enthalten. Mit anderen Worten kann die Transfektion/Transformation eine stabile oder transiente Transfektion/Transformation sein. Transfektions- und Transformations-Protokolle sind dem Fachmann bekannt (Chan and Cohen. 1979. High Frequency Transformation of Bacillus subtilis Protoplasts by Palsmid DNA. Mol Gen Genet. 168(1):111-5; Kieser et al.. 2000. Practical Streptomyces Genetics. The John Innes Foundation Norwich.; Sambrook et al.. 1989. Molecular Cloning. A Laboratory Manual. In: second ed.. Cold Spring Harbor Laboratory Press. Cold Spring Harbor. NY.; Irani and Rowe. 1997. Enhancement of transformation in Pseudomonas aeruginosa PAO1 by Mg2+ and heat. Biotechniques 22: 54-56; Balbas, P. und Bolivar, F. (1990), Design and construction of expression plasmid vectors in E.coli, Methods Enzymol. 185, 14-37; Rodriguez, R.L. und Denhardt, D. T (eds) (1988), Vectors: a survey of molecular cloning vectors and their uses, 205-225, Butterworth, Stoneham). Bezüglich der allgemeinen Vorgehensweise (PCR, Klonierung, Expression etc.) sei auch auf folgende Literatur und das dort zitierte verwiesen: Universal GenomeWalker™ Kit User Manual, Clontech, 3/2000; Triglia T.; Peterson, M. G. und Kemp, D.J. (1988), A procedure for in vitro amplification of DNA segments that lie outside the boundaries of known sequences, Nucleic Acids Res. 16, 8186.

Vorzugsweise ist der Wirt ein rekombinanter Mikroorganismus prokaryotischen Ursprungs. Geeignete Wirtszellen schließen Zellen von einzelligen Mikroorganismen wie bakterielle Zellen ein. Als Mikroorganismen sind diesbezüglich Prokaryonten, wie E. coli, Bacillus subtilis, zu nennen. Ferner können Bakterien der Genera/Spezies Lactobacillus, Bacillus, Rhodococus, Campylobacter, Caulobacter, Mycobacterium, Streptomyces, Neisseria, Ralstonia, Pseudomonas, sowie Agrobacterium für die Expression der erfindungsgemäßen Nukleinsäuresequenzen eingesetzt werden. Vorzugsweise sind E. coli-Stämme für diesen Zweck zu benutzen. Ganz besonders bevorzugt sind: E. coli XL1 Blue, NM 522, JM101, JM109, JM105, RR1, DH5α, TOP 10-, HB101, BL21 codon plus, BL21 (DE3) codon plus, BL21, Rosetta, Rosetta-gami, MM294, W3110, DSM14459 (EP1444367), Origami. Entsprechende Stämme sind im Stand der Technik verfügbar und können, zumindest teilweise, über die internationalen Hinterlegungsstellen wie die ATCC oder die DMSZ bezogen werden. Gleichfalls können Eukaryonten, wie Säugerzellen, Insektenzellen oder Pflanzenzellen oder Organismen wie z.B. Hefen wie Hansenula polymorpha, Pichia sp., Saccharomyces cerevisiae, bzw. Pilze, wie z.B. Aspergillus sp., für die rekombinante Herstellung der Polypeptide eingesetzt werden. Geeignete eukaryontische Zellen schließen CHO-Zellen, HeLa-Zellen und andere ein. Viele dieser Zellen sind über Hinterlegungsstellen wie die ATCC oder die DMSZ erhältlich.

Ferner kann die rekombinante Herstellung der erfindungsgemäßen Polypeptide in einem nichtmenschlichen Wirt erfolgen. Der nicht-menschliche Wirt kann eine Zelle oder ein mehr- bis vielzelliger Organismus sein. Geeignete vielzellige Organismen schließen in der Molekularbiologie geläufige Modellsysteme wie Drosophila melanogaster, Zebrafisch oder C. elegans ein. Transgene nicht-menschliche Tiere können nach im Stand der Technik bekannten Verfahren hergestellt werden. Das transgene nicht-menschliche Tier kann bevorzugt verschiedene genetische Konstitutionen aufweisen. Es kann (i) das Gen einer erfindungsgemäßen Nukleinsäuresequenz konstitutiv oder induzierbar überexprimieren, (ii) das endogene Gen einer erfindungsgemäßen Nukleinsäuresequenz in inaktivierter Form enthalten, (iii) das endogene Gen einer erfindungsgemäßen Nukleinsäuresequenz vollständig oder teilweise durch ein mutiertes Gen einer erfindungsgemäßen Nukleinsäuresequenz ersetzt enthalten, (iv) eine konditionale und gewebsspezifische Überexpression oder Unterexpression des Gens einer erfindungsgemäßen Nukleinsäuresequenz aufweisen oder (v) einen konditionalen und gewebsspezifischen Knock-out des Gens einer erfindungsgemäßen Nukleinsäuresequenz aufweisen.

Vorzugsweise enthält das transgene Tier zusätzlich ein exogenes Gen einer erfindungsgemäßen Nukleinsäuresequenz unter Kontrolle eines die Überexpression erlaubenden Promotors. Alternativ kann das endogene Gen einer erfindungsgemäßen Nukleinsäuresequenz durch Aktivierung oder/und Austausch des eigenen Promotors überexprimiert werden. Vorzugsweise weist der endogene Promotor des Gens einer erfindungsgemäßen Nukleinsäuresequenz eine genetische Veränderung auf, die zu einer erhöhten Expression des Gens führt. Die genetische Veränderung des endogenen Promotors umfasst dabei sowohl eine Mutation einzelner Basen als auch Deletions- und Insertionsmutationen. In einer besonders bevorzugten Ausführungsform des Wirts ist dieser ein transgener Nager, vorzugsweise eine transgene Maus, ein transgenes Kaninchen, eine transgene Ratte, oder ein transgenes Schaf, eine transgene Kuh, eine transgene Ziege oder ein transgenes Schwein. Mäuse haben gegenüber anderen Tieren zahlreiche Vorteile. Sie sind leicht zu halten und ihre Physiologie gilt als Modellsystem für die des Menschen. Die Herstellung solcher Gen-manipulierten Tiere ist dem Fachmann hinreichend bekannt und wird nach üblichen Verfahren durchgeführt (s.h. z.B. Hogan, B., Beddington, R., Costantini, F. und Lacy, E. (1994), Manipulating the Mouse-Embryo; A Laboratory Manual, 2. Aufl., Cold Spring Harbor Laboratory, Cold Spring Harbor, NY; WO91/08216). Alternativ oder zusätzlich können auch Zellkultursysteme, insbesondere humane Zellkultursysteme, für die Anwendungen eingesetzt werden, die für das nicht-menschliche transgene Tier beschrieben sind.

Eine weitere Ausgestaltung der Erfindung bezieht sich auf komplette Gene, welche die erfindungsgemäßen Nukleinsäuren aufweisen. Unter Gen wird anmeldungsgemäß ein Abschnitt auf molekularer Ebene verstanden, der prinzipiell aus zwei unterschiedlichen Bereichen bestehen kann:
● einem DNA-Abschnitt, von dem durch Transkription eine einzelsträngige RNA-Kopie hergestellt wird
● allen zusätzlichen DNA-Abschnitten, die an der Regulation dieses Kopiervorgangs beteiligt sind.

Genauere Definitionen können der Seite: http://de.wikipedia.org/wiki/Gen entnommen werden.

Die codierenden Nukleinsäuresequenzen können in herkömmliche Plasmide/Vektoren kloniert und nach Transfektion von Mikroorganismen oder anderen Wirtszellen mit solchen Vektoren in Zellkultur exprimiert werden. Als Plasmide oder Vektoren kommen im Prinzip alle dem Fachmann für diesen Zweck zur Verfügung stehende Ausführungsformen in Frage. Derartige Plasmide und Vektoren können z. B. von Studier und Mitarbeiter (Studier, W. F.; Rosenberg A. H.; Dunn J. J.; Dubendroff J. W.; , Use of the T7 RNA polymerase to direct expression of cloned genes, Methods Enzymol. 1990, 185, 61-89) oder den Broschüren der Firmen Novagen, Promega, New England Biolabs, Clontech oder Gibco BRL entnommen werden. Weiter bevorzugte Plasmide und Vektoren können gefunden werden in: Glover, D. M. (1985), DNA cloning: a practical approach, Vol. I-III, IRL Press Ltd., Oxford; Rodriguez, R.L. und Denhardt, D. T (eds) (1988), Vectors: a survey of molecular cloning vectors and their uses, 179-204, Butterworth, Stoneham; Goeddel, D. V., Systems for heterologous gene expression, Methods Enzymol. 1990, 185, 3-7; Sambrook, J.; Fritsch, E. F. und Maniatis, T. (1989), Molecular cloning: a laboratory manual, 2nd ed., Cold Spring Harbor Laboratory Press, New York. Plasmide, mit denen die erfindungsgemäße Nukleinsäuresequenz oder ein sie enthaltendes Genkonstrukt in ganz bevorzugter Weise in den Wirtsorganismus kloniert werden kann, sind: pUC18 (Roche Biochemicals), pKK-177-3H (Roche Biochemicals), pBTac2 (Roche Biochemicals), pKK223-3 (Amersham Pharmacia Biotech), pKK-233-3 (Stratagene) oder pET (Novagen). Geeignete Vektoren sind z. B. auch pET-21a(+) für E. coli, aber auch andere Expressionsvektoren für prokaryontische Einzeller sowie Vektoren für Eukaryoten können verwendet werden. Als geeignete Vektoren für Hefen haben sich z. B. der pREP-Vektor oder der pINT-Vektor erwiesen. Für die Expression in Insektenzellen sind z. B. Baculovirus-Vektoren wie in EP127839 oder EP549721 offenbart, und für die Expression in Säugerzellen sind z. B. SV40-Vektoren geeignet, welche allgemein erhältlich sind. Besonders bevorzugt sind Vektoren für einzellige eukaryontische Organismen, insbesondere aus der Gruppe der pET-Vektoren für die Transformation von E. coli-Zellen, insbesondere E. coli Origami.

In einer besonders bevorzugten Ausführungsform ist die in den Vektor eingeschleuste erfindungsgemäße Nukleinsäuresequenz darüber hinaus mit einem von dem Vektor bereitgestellten Histidin-Tag fusioniert. Bevorzugt wird die eingeschleuste Nukleinsäuresequenz so in den bevorzugten pET-Vektor kloniert, dass die Transkription unter der Kontrolle des im Vektor vorhandenen IPTG-regulierbaren Promotors steht. Alternativ werden auch Rhamnoseregulierbare Promotoren bevorzugt eingesetzt.

Die Vektoren können neben den üblichen Markern, wie z. B. Antibiotika-Resistenzgenen, weitere funktionelle Nukleotidsequenzen zur Regulation, insbesondere zur Repression oder Induktion der Expression des ADH-Gens und/oder eines Reportergens enthalten. Als Promotoren werden bevorzugt regulierbare schwache Promotoren, wie z. B. der rha-Promotor oder der nmt1-Promotor, oder regulierbare starke Promotoren, wie z. B. der lac-, ara-, lambda-, pL-, T7- oder T3-Promotor, benutzt. Die codierenden DNA-Fragmente müssen in den Vektoren von einem Promotor aus transkribierbar sein. Weitere bewährte Promotoren sind z. B. der Baculovirus-Polyhedrin-Promotor für die Expression in Insektenzellen (s. z. B. EP127839) oder der frühe SV40-Promotor oder LTR-Promotoren z. B. von MMTV (Mouse Mammary Tumour Virus; Lee et al. (1981) Nature, 294 (5838), 228-232).

Die erfindungsgemäßen Gene, Vektoren/Plasmide können demnach weitere funktionale Sequenzbereiche, wie z. B. einen Replikationsstartpunkt, Operatoren, oder Terminationssignale enthalten.

In einer besonders vorteilhaften Ausführungsform bezieht sich die vorliegende Erfindung auf rec-Mikroorganismen, die neben der erfindungsgemäßen Nukleinsäuresequenz auch ein oder mehrere klonierte Chaperon-Gene enthalten. Als bevorzugte Chaperone kommen GroEL und GroES vorzugsweise in einem E. coli Origami Stamm in Frage. Überraschenderweise wurde in Gegenwart dieser beiden Faltungshelferproteine eine Expression des aktiven Enzyms erreicht, obwohl andere alternative Chaperonsysteme, wie z.B. die E. coli-eigenen Chaperone, induziert durch Ethanol Zugabe oder andere coexprimierte Chaperone, wie DnaK, DnaJ und GrpE nicht zum Erfolg führen (DE 10061864).

Für den Fachmann überraschend ist, dass eine gleichwertige Coexpression der Chaperonsysteme Dnak, DnaJ, GrpE und GroEL, GroES oder die Coexpression von GroEL oder GroES allein zusammen mit der Schweineleberesterase in E. coli Origami nur zu einer Expression in Form von Inclusion Bodies führt und nicht zu einer nachweisbaren Aktivität in E. coli Rohzellextrakt. Daher ist es in jedem Falle bevorzugt, dass das Chaperone-System GroEL /GroES das bevorzugt induzierte / exprimierte System ist, auch wenn andere Chaperone-Systeme gleichzeitig in dem Wirtsorganismus vorliegen.

Die funktionelle Expression von eukaryotischen Proteinen in E. coli stellt eine große Herausforderung dar, insbesondere falls es sich um posttranslational glykosylierte Proteine handelt. Im Falle der rekombinanten Expression der Schweineleberesterase in E. coli gleicht der Einsatz des speziellen Chaperonsystems GroEL, GroES die fehlende posttranslatorische Glykosylierung offenbar aus. Bzgl. dieser Ausgestaltung und deren Ausführung wird auf die DE102006031600 verwiesen.

In einer nächsten Ausgestaltung richtet sich die vorliegende Erfindung auf die Verwendung der erfindungsgemäßen (rec-)Polypeptide zur Herstellung von enantiomerenangereicherten Alkoholen, Carbonsäuren bzw. Estern, insbesondere aus Mesoformen der eben genannten Verbindungen wie z.B. ggf. substituierten Dicarbonsäureestern wie Malonsäuredieestern, .

Möglich ist die Verwendung der Enzyme in immobilisierter Form (Sharma B. P.; Bailey L. F. und Messing R. A. (1982), Immobilisierte Biomaterialien - Techniken und Anwendungen, Angew. Chem. 94, 836-852). Vorteilhafterweise erfolgt die Immobilisierung durch Lyophilisation (Paradkar, V. M.; Dordick, J. S. (1994), Aqueous-Like Activity of α-Chymotrypsin Dissolved in Nearly Anhydrous Organic Solvents, J. Am. Chem. Soc. 116, 5009-5010; Mori, T.; Okahata, Y. (1997), A variety of lipi-coated glycoside hydrolases as effective glycosyl transfer catalysts in homogeneous organic solvents, Tetrahedron Lett. 38, 1971-1974; Otamiri, M.; Adlercreutz, P.; Matthiasson, B. (1992), Complex formation between chymotrypsin and ethyl cellulose as a means to solubilize the enzyme in active form in toluene, Biocatalysis 6, 291-305). Ganz besonders bevorzugt ist die Lyophilisation in Gegenwart von oberflächenaktiven Substanzen, wie Aerosol OT oder Polyvinylpyrrolidon oder Polyethylenglycol (PEG) oder Brij 52 (Diethylenglycol-mono-cetylether) (Kamiya, N.; Okazaki, S.-Y.; Goto, M. (1997), Surfactanthorseradish peroxidase complex catalytically active in anhydrous benzene, Biotechnol. Tech. 11, 375-378).

Äußerst bevorzugt ist die Immobilisierung an Eupergit®, insbesondere Eupergit C® und Eupergit 250L® (Röhm) (als Übersicht, siehe: E. Katchalski-Katzir, D. M. Kraemer, J. Mol. Catal. B: Enzym. 2000, 10, 157). Gleichfalls bevorzugt ist die Immobilisierung an Ni-NTA in Kombination mit dem durch Anhängen eines His-Tags (Hexa-Histidin) veränderten Polypeptid (Petty, K.J. (1996), Metal-chelate affinity chromatography In: Ausubel, F.M. et al. eds. Current Protocols in Molecular Biology, Vol. 2, New York: John Wiley and Sons).

Die Verwendung als CLECs ist ebenfalls denkbar (St. Clair, N.; Wang, Y.-F.; Margolin, A. L. (2000), Cofactor-bound cross-linked enzyme crystals (CLEC) of alcohol dehydrogenase, Angew. Chem. Int. Ed. 39, 380-383).

Durch diese Maßnahmen kann es gelingen, aus (rec-)Polypeptiden, welche durch organische Solventien instabil werden, solche zu generieren, die in Gemischen von wässrigen und organischen Lösungsmitteln bzw. ganz in Organik arbeiten können.

Für die Umsetzung von Estern oder Carbonsäuren und Alkoholen mit den erfindungsgemäßen Polypeptiden geht man vorzugsweise wie folgt vor. Die Polypeptide werden in der gewünschte Form (frei, immob., in Wirtsorganismen oder in beliebig hoch aufgeschlossener Form) in das entsprechende Medium, vorzugsweise die wässrige Lösung gegeben. Unter Einhaltung der optimalen Temperatur- bzw. des optimalen pH-Wertbereiches wird das Substrat zu dieser Mischung gegeben. Nach der erfolgten Umsetzung kann der gewonnene Alkohol bzw. Ester nach dem Fachmann bekannten Verfahren (Kristallisation, Extraktion, Chromatographie) aus der Reaktionsmischung isoliert werden.

Die enzymatische Umsetzung von Estern oder Carbonsäuren und Alkoholen zu enantiomerenangereicherten Alkoholen, Carbonsäuren bzw. Estern mittels Esterasen ist dem Fachmann grundsätzlich bekannt (siehe eingangs angegebene Literaturstellen; Schema 2). Insbesondere interessant sind in diesem Zusammenhang die Umsetzungen von Mesoformen der eben genannten Derivate. Hier wirkt sich die erfindungsgemäße Umsetzung dahingehend positiv aus, dass ein enantiomerenangereichertes Produkt zu 100% Ausbeute erzielt werden kann, während bei normalen Racematspaltungen nur max. 50% Ausbeute der jeweiligen Enantiomere erhalten werden können. So lassen sich z.B. bei Einsatz bestimmter substituierter Malonsäurediester enantiomerenangereicherte Produkte erhalten, die wertvolle Intermediate in der chemischen Synthese sind. Aus ggf. N-geschützten Aminomalonsäurediestern lassen sich so in effizienter und einfacher Art und Weise die entsprechenden enantiomerenangereicherten unsymmetrischen Aminocarbonsäuremonester herstellen. Weiterhin interessant ist die in folgendem Schema 1 dargestellte Reaktionsfolge.

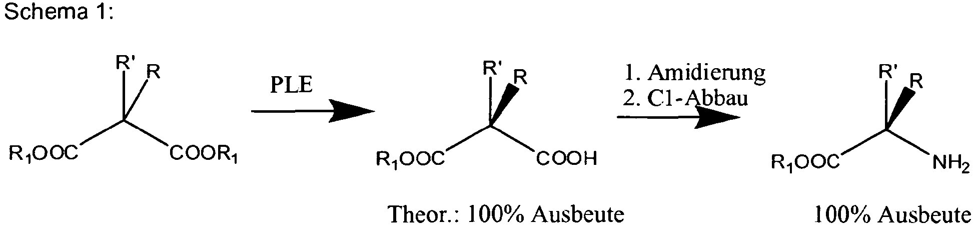

Die für diese Reaktion in Frage kommenden Reste können unten stehender Aufstellung entnommen werden.

Die Maßnahmen zur Amidierung und zum C1-Abbau sind dem Fachmann hinlänglich bekannt (Organikum, VEB Deutscher Verlag der Wissenschaften, Berlin 1986, S. 388ff, S. 571ff).

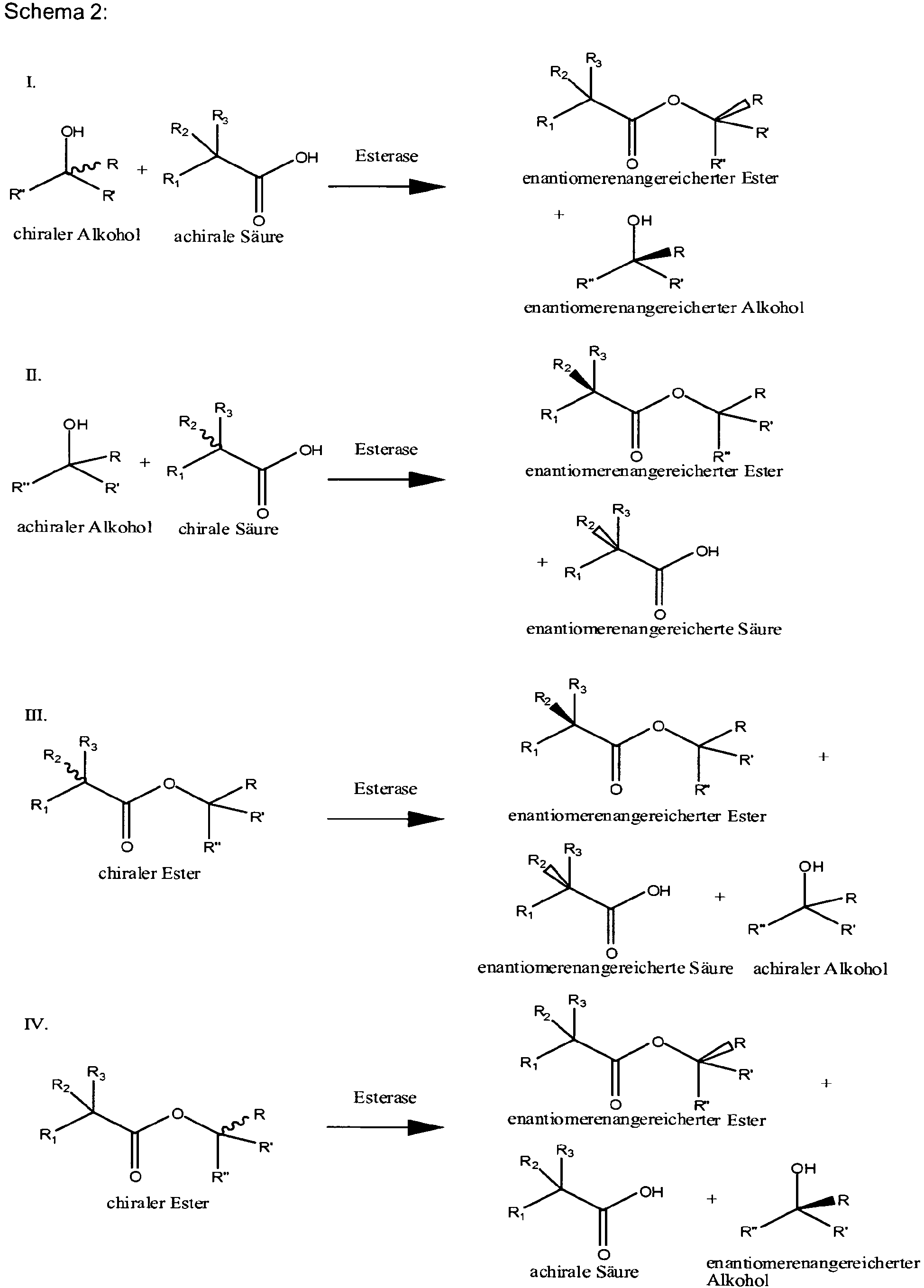

Beispiele für enantiomerenangereicherte Alkohole, die entsprechend I. oder VI. aus den entsprechenden chiralen Estern hergestellt werden können oder mit deren Hilfe enantiomerenangereicherte Ester hergestellt werden können, sind dem Fachmann ebenfalls geläufig. Beispielsweise lassen diese sich unter folgende allgemeine Formel subsumieren, 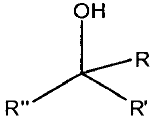 in der R, R' und R" verschieden voneinander sind, insbesondere H, (C₁-C₈)-Alkyl, (C₁-C₈)-Alkoxy, HO-(C₁-C₈)-Alkyl, (C₂-C₈)-Alkoxyalkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₈)-Heteroaryl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl oder R und R' und/oder R und R" und/oder R' und R" bilden eine (C₃-C₅)-Alkylenbrücke.

Beispiele für enantiomerenangereichte Ester bzw. Säuren können folgenden allgemeinen Formeln zugeordnet werden, 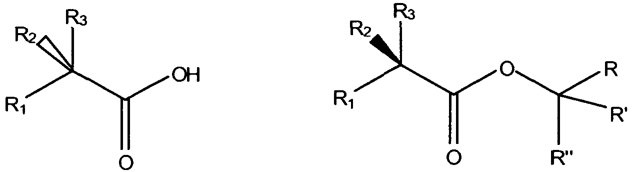 in der R, R' und R" gleich oder verschieden voneinander sind, insbesondere H, (C₁-C₈)-Alkyl, (C₁-C₈)-Alkoxy, HO-(C₁-C₈)-Alkyl, (C₂-C₈)-Alkoxyalkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₈)-Heteroaryl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl oder R und R' oder R und R" oder R' und R" bilden eine (C₃-C₅)-Alkylenbrücke und R₁, R₂ und R₃ verschieden voneinander sind, insbesondere H, (C₁-C₈)-Alkyl, (C₁-C₈)-Alkoxy, HO-(C₁-C₈)-Alkyl, (C₂-C₈)-Alkoxyalkyl, Cyclopentadienyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₈)-Heteroaryl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl oder R₁ und R₂ und/oder R₁ und R₃ und/oder R₂ und R₃ bilden eine (C₃-C₅)-Alkylenbrücke.

Für die erfindungsgemäße Verwendung eignen sich wässrige Lösungsmittel, die entsprechend gepuffert sind. Es ist jedoch auch möglich, die Reaktion mit einem pH-Stat-Automaten [Firma:Schott AG, Mainz, Marke TitroLine alpha] zu betreiben.

Bevorzugt wird die Umsetzung bei einer Temperatur zwischen 0°C und 85°C, besonders bevorzugt zwischen 30 und 80°C, ganz besonderes bevorzugt um 50°C ausgeführt. Auch bei dem pH-Wert der Reaktion ist der Fachmann frei in der Wahl, wobei die Reaktion sowohl bei einem festen pH-Wert, als auch unter Variation des pH-Werts in einem pH-Intervall durchgeführt werden kann. Der pH-Wert wird insbesondere unter Berücksichtigung eines optimalen Reaktionsergebnisses gemäß der vorliegenden Aufgabe gewählt. Bevorzugt wird die Reaktion bei einem pH-Wert durchgeführt, der bei pH 5 bis 9, bevorzugt pH 6 bis 8 und besonders bevorzugt pH 6.5 bis 7.5 liegt.

Für die Anwendung kann das betrachtete Polypeptid - wie weiter oben schon angesprochen - in nativer Form als homogen aufgereinigte Verbindungen oder als rekombinant hergestelltes Enzym verwendet werden. Weiterhin kann das (rec-)Polypeptid auch als Bestandteil eines intakten Gastorganismus eingesetzt werden oder in Verbindung mit der aufgeschlossenen und beliebig hoch aufgereinigten Zellmasse des Wirtsorganismus.

Erfolgt die Umsetzung des eingesetzten Substrates zu dem gewünschten Produkt in Zellkultur, z.B. unter Einsatz eines geeigneten Wirtes, wird je nach verwendetem Wirtsorganismus oder der verwendeten Zellkultur ein geeignetes Nährmedium verwendet. Die für die Wirtszellen geeigneten Medien sind allgemein bekannt und kommerziell erhältlich. Den Zellkulturen können außerdem übliche Zusätze zugegeben werden, wie z.B. Antibiotika, wachstumsfördernde Mittel, wie z.B. Seren (fötales Kälberserum usw.) und ähnliche bekannte Zusatzstoffe.

Weitere optimale Reaktionsbedingungen können der DE102006031600 entnommen werden.

Eine weitere Anwendung betrifft die Herstellung eines Polypeptids mit gegenüber dem Polypeptid der SEQ. ID. NO: 2 verbesserter Aktivität und/oder Selektivität und/oder Stabilität, durch
i) Mutagenese der erfindungsgemäßen Nukleinsäure, bevorzugt die der Seq. 3, 5, 7, 9,
ii) Klonierung der aus i) erhältlichen Nukleinsäuresequenz in einen geeigneten Vektor mit anschließender Transformation in ein geeignetes Expressionssystem und
iii) Detektion und Isolierung des maßgeblichen Polypeptids mit verbesserter Aktivität und/oder Selektivität und/oder Stabilität.

Die Vorgehensweise zur Verbesserung der erfindungsgemäßen Nukleinsäuresequenzen bzw. der durch sie codierten Polypeptide durch Mutagenese-Methoden ist dem Fachmann hinlänglich bekannt. Als Mutagenese-Methoden kommen alle dem Fachmann für diesen Zweck zur Verfügung stehenden Methoden in Frage. Insbesondere sind dies die Sättigungsmutagenese, die Random-Mutagenesis, in vitro-Rekombinations-Methoden sowie Site-Directed-Mutagenesis (Eigen, M. und Gardiner, W., Evolutionary molecular engineering based on RNA replication, Pure Appl. Chem. 1984, 56, 967-978; Chen, K. und Arnold, F., Enzyme engineering for nonaqueous solvents: random mutagenesis to enhance activity of subtilisin E in polar organic media. Bio/Technology 1991, 9, 1073-1077; Horwitz, M. und Loeb, L., Promoters Selected From Random DNA-Sequences, Proc Natl Acad Sci USA 83, 1986, 7405-7409; Dube, D. und L. Loeb, Mutants Generated By The Insertion Of Random Oligonucleotides Into The Active-Site Of The Beta-Lactamase Gene, Biochemistry 1989, 28, 5703-5707; Stemmer, P.C., Rapid evolution of a protein in vitro by DNA shuffling, Nature 1994, 370, 389-391 und Stemmer, P.C., DNA shuffling by random fragmentation and reassembly: In vitro recombination for molecular evolution. Proc Natl Acad Sci USA 91, 1994, 10747-10751).

Die erhaltenen neuen Nukleinsäuresequenzen werden nach den unten angegebenen Methoden in einen Wirtsorgansmus (Lit. s.u.) kloniert und die so exprimierten Polypeptide mit geeigneten Screening-Methoden detektiert und anschließend isoliert. Zur Detektion sind grundlegend alle möglichen Nachweisreaktionen für die mit diesem Polypeptid gebildeten Moleküle geeignet. Insbesondere dienen dazu photometrische Tests über gebildetes oder verbrauchtes NADH, HPLC- oder GC-Methoden zum Nachweis der mit diesem Enzym gebildeten Alkohole. Zum Nachweis neuer, durch gentechnische Methoden veränderter Polypeptide sind zudem auch gelelektrophoretische Nachweismethoden oder Nachweismethoden mittels Antikörpern geeignet.

Unter optisch angereicherten (enantiomerenangereicherten, Enantiomer-angereicherten?) Verbindungen wird im Rahmen der Erfindung das Vorliegen einer optischen Antipode im Gemisch mit der anderen in >50 mol-% verstanden.

Unter dem Begriff Nukleinsäuresequenzen werden alle Arten von einzelsträngiger oder doppelsträngiger DNA als auch RNA oder Gemische derselben subsumiert. Die erfindungsgemäße Nukleinsäuresequenz kann demnach ein DNA- oder ein RNA-Molekül sein. Bevorzugt ist, dass das Nukleinsäuremolekül ein cDNA- oder ein mRNA-Molekül ist. Erfindungsgemäß kann das DNA-Molekül des Weiteren ein genomisches DNA-Molekül sein. Ferner umfasst von der Erfindung sind Ausführungsformen, in denen das DNA-Molekül ein PNA-Molekül oder ein anderes Derivat eines DNA-Moleküls ist.

Der Begriff "komplementär" bedeutet erfindungsgemäß, dass sich die Komplementarität über den gesamten Bereich des erfindungsgemäßen Nukleinsäuremoleküls ohne Lücken erstreckt. Mit anderen Worten ist es erfindungsgemäß bevorzugt, dass die Komplementarität sich zu 100% über den gesamten Bereich der erfindungsgemäßen Sequenz, d.h. vom dargestellten 5'-Ende bis zum dargestellten 3'-Ende erstreckt.

Die Verbesserung der Aktivität und/oder Selektivität und/oder Stabilität bedeutet erfindungsgemäß, dass die Polypeptide aktiver und/oder selektiver bzw. unter den verwendeten Reaktionsbedingungen stabiler sind. Während die Aktivität und die Stabilität der Enzyme für die technische Anwendung naturgemäß möglichst hoch sein sollte, ist in Bezug auf die Selektivität dann von einer Verbesserung die Rede, wenn die Substratselektivität abnimmt, die Enantioselektivität der Enzyme jedoch gesteigert ist. Für den in diesem Zusammenhang gebrauchten Ausdruck nicht wesentlich reduziert gilt mutatis mutandis das Selbe.

Von den beanspruchten Proteinsequenzen und den Nukleinsäuresequenzen werden erfindungsgemäß auch solche Sequenzen umfasst, die eine Homologie (exclusive der natürlichen Degeneration) größer als 97 %, bevorzugt größer als 97,5 %, 98 % oder 98,5 %, mehr bevorzugt größer als 99 % oder 99,5 % zu einer dieser Sequenzen aufweisen, sofern die Wirkungsweise bzw. Zweck einer solchen Sequenz erhalten bleibt. Der Ausdruck "Homologie" (oder Identität) wie hierin verwendet, kann durch die Gleichung H (%) = [1 - V/X] x 100 definiert werden, worin H Homologie bedeutet, X die Gesamtzahl an Nukleobasen/Aminosäuren der Vergleichssequenz ist und V die Anzahl an unterschiedlichen Nukleobasen/Aminosäuren der zu betrachtenden Sequenz bezogen auf die Vergleichssequenz ist. Auf jeden Fall sind mit dem Begriff Nukleinsäuresequenzen, welche für Polypeptide codieren, alle Sequenzen umfasst, die nach Maßgabe der Degeneration des genetischen Codes möglich erscheinen.

Der Ausdruck "unter stringenten Bedingungen" wird hierin wie bei Sambrook et al. (Sambrook, J.; Fritsch, E. F. und Maniatis, T. (1989), Molecular cloning: a laboratory manual, 2nd ed., Cold Spring Harbor Laboratory Press, New York) beschrieben, verstanden. Bevorzugt liegt eine stringente Hybridisierung gemäß der vorliegenden Erfindung vor, wenn nach Waschen für eine Stunde mit 1 x SSC (150 mM Natriumchlorid, 15 mM Natriumcitrat, pH 7.0) und 0,1 % SDS (Natriumdodecylsulfat) bei 50 °C, bevorzugt bei 55 °C, mehr bevorzugt bei 62 °C und am meisten bevorzugt bei 68 °C und mehr bevorzugt für 1 Stunde mit 0,2 x SSC und 0,1 % SDS bei 50 °C, bevorzugter bei 55 °C, mehr bevorzugt bei 62 °C und am meisten bevorzugt bei 68-°C noch ein positives Hybridisierungssignal beobachtet wird.

Als (C₁-C₈)-Alkylreste sind anzusehen Methyl, Ethyl, *n*-Propyl, Isopropyl, *n*-Butyl, Isobutyl, *sec*-Butyl, *tert*-Butyl, Pentyl, Hexyl, Heptyl oder Octyl samt aller ihrer Bindungsisomeren.

Ein (C₁-C₂₀)-Alkylrest ist im Rahmen der erfindungsgemäßen Definition ein entsprechender Rest mit 1 bis max. 20 C-Atomen.

Ein (C₃-C₂₀)-Alkylrest ist im Rahmen der erfindungsgemäßen Definition ein entsprechender Rest mit 3 bis max. 20 C-Atomen.

Der Rest (C₁-C₈)-Alkoxy entspricht dem Rest (C₁-C₈)-Alkyl mit der Maßgabe, dass dieser über ein Sauerstoffatom gebunden ist.

Als (C₂-C₈)-Alkoxyalkyl sind Reste gemeint, bei denen die Alkylkette durch mindestens eine Sauerstofff(?)unktion unterbrochen ist, wobei nicht zwei Sauerstoffatome miteinander verbunden sein können. Die Anzahl der Kohlenstoffatome gibt die Gesamtzahl der im Rest enthaltenen Kohlenstoffatome an.

Eine (C₃-C₅)-Alkylenbrücke ist eine Kohlenstoffkette mit drei bis fünf C-Atomen, wobei diese Kette über zwei verschiedene C-Atome an das betrachtete Molekül gebunden ist.

Die eben beschriebenen Reste können einfach oder mehrfach mit Halogenen und/oder (C₁-C₈)-Alkoxycarbonyl und/oder N-, O-, P-, S-, Si-atomhaltigen Resten substituiert sein. Dies sind insbesondere Alkylreste der oben genannten Art, welche eines oder mehrere dieser Heteroatome in ihrer Kette aufweisen bzw. welche über eines dieser Heteroatome an das Molekül gebunden sind.

Unter (C₃-C₈)-Cycloalkyl versteht man Cyclopropyl, Cyclobutyl, Cyclopentyl, Cyclohexyl bzw. Cycloheptylreste etc. Diese können mit einem oder mehreren Halogenen und/oder N-, O-, P-, S-, Si-atomhaltige Reste substituiert sein und/oder N-, O-, P-, S-Atome im Ring aufweisen, wie z. B. 1-, 2-, 3-, 4-Piperidyl, 1-, 2-, 3-Pyrrolidinyl, 2-, 3-Tetrahydrofuryl, 2-, 3-, 4-Morpholinyl.

Ein (C₃-C₈)-Cycloalkyl-(C₁-C₈)-Alkylrest bezeichnet einen wie oben dargestellten Cycloalkylrest, welcher über einen wie oben angegebenen Alkylrest an das Molekül gebunden ist.

(C₁-C₈)-Alkoxycarbonyl bedeutet im Rahmen der Erfindung einen wie oben definierten Alkylrest mit max. 8 C-Atomen, welcher über eine O(C=O)-Funktion gebunden ist.

(C₁-C₈)-Acyloxy bedeutet im Rahmen der Erfindung einen wie oben definierten Alkylrest mit max. 8 C-Atomen, welcher über eine (C=O)O-Funktion gebunden ist.

(C₁-C₈)-Acyl bedeutet im Rahmen der Erfindung einen wie oben definierten Alkylrest mit max. 8 C-Atomen, welcher über eine (C=O)-Funktion gebunden ist.

Unter einem (C₆-C₁₈)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl-, Biphenylreste oder an das betreffende Molekül annelierte Systeme der vorbeschriebenen Art, wie z.B. Indenylsysteme, welche ggf. mit ((C₁-C₈)-Alkyl, (C₁-C₈)-Alkoxy, (C₂-C₈)-Alkoxyalkyl, NH(C₁-C₈)-Alkyl, N((C₁-C₈)-Alkyl)₂, OH, O(C₁-C₈)-Alkyl, NO₂, NH(C₁-C₈)-Acyl, N((C₁-C₈)-Acyl)₂, F, Cl, CF₃, (C₁-C₈)-Acyl, (C₁-C₈)-Acyloxy, (C₇-C₁₉)-Aralkylrest, (C₄-C₁₉)-Heteroaralkyl substituiert sein können.

Ein (C₇-C₁₉)-Aralkylrest ist ein über einen (C₁-C₈)-Alkylrest an das Molekül gebundener (C₆-C₁₈)-Arylrest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Reste angesehen, wie 1-, 2-, 3-Furyl, wie 1-, 2-, 3-Pyrrolyl, 1-, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, Acridinyl, Chinolinyl, Phenanthridinyl, 2-, 4-, 5-, 6-Pyrimidinyl. Die Heteroaromaten können in gleicher Weise wie die oben genannten (C₆-C₁₈)-Arylreste substituiert sein.

Unter einem (C₄-C₁₉)-Heteroaralkyl wird ein dem (C₇-C₁₉)-Aralkylrest entsprechendes heteroaromatisches System verstanden.

Als Halogene (Hal) kommen Fluor, Chlor, Brom und Iod in Frage.

Unter dem Begriff wässriges Lösungsmittel wird Wasser oder ein hauptsächlich aus Wasser bestehendes Lösungsmittelgemisch mit wasserlöslichen organischen Lösungsmitteln wie z.B. Alkoholen, insbesondere Methanol oder Ethanol, oder Ethern, wie THF oder Dioxan, oder anderen Colsolveintien wie DMSO verstanden.

Die in dieser Schrift genannten Literaturstellen gelten als von der Offenbarung mitumfasst.

Die dargestellten Proteinsequenzen im Sequenzprotokoll enthalten am C-terminus zusätzlich eine His-Tag- und eine Linker-Sequenz. Die eigentlichen Proteinsequenzen, welche die aktiven γPLEs darstellen, sind daher die im Sequenzuprotokoll dargestellten und am C-terminus um 21 Aminosäuren verkürzten Sequenzen. Für die Nukleinsäuresequenzen, die für diese Proteinsequenzen codieren, gilt entsprechendes.

### Methoden:

### Isolierung der mRNA und Synthese der cDNA

Gewebe von frischer Schweineleber (0,1 g) wurde mit Trizol® Reagenz (TRIzol® Plus RNA Purification Kit, Invitrogen, CA, USA) versetzt, homogenisiert (10 min bei RT; Ultraturrax T25, IKA-Labortechnik) und die RNA nach den Angaben des Herstellers isoliert. Die RNA Konzentration wurde spektrophotometrisch bestimmt. Die cDNA-Synthese erfolgte durch RT-PCR unter Verwendung von oligo(dT)15 Primern und MMLV Reverser Transkriptase mit RNase H Aktivität (Promega, Madison, WI, USA) nach dem Protokoll des Herstellers.

### Amplifikation und Klonierung von PLE Genen

Das Produkt der RT-PCR wurde eingesetzt für die Amplifikation von PLE Genen mit zwei Genspezifischen Primern, die auf der Sequenz der γPLE basieren (5'-CACCCAT*ATG*GGGCAGCCAGCCTCGC-3' (Seq. ID Nr.: 11), mit kursiv markierter Restriktionsschnittstelle für Ndel und 5'-CCG*CTCGAG*TCACTTTATCTTGGGTGGCTTCTTTGC-3' (Seq. ID Nr.: 12), mit kursiv markierter Restriktionsschnittstelle für Xhol; unterstrichen sind Start- und Stopp-Codon). Der forward-Primer enthält zudem an seinem 5'-Ende die Basen CACC, die das anschließende Klonieren in einen TOPO Vektor ermöglichen (s. unten). Diese Primer eliminieren bereits die im ursprünglichen Schweinegen anhängende N-terminale 18 Aminosäuren lange Signalsequenz sowie das 4 Aminosäuren lange C-terminale ER (Endoplasmatisches Retikulum)-Retentionssignal, was die nachfolgende Expression in E.coli erleichtert (Lange, S, et. al., ChemBioChem (2001), 2, 576 - 582). Die PCR wurde in einem Thermocycler (Techne Progene, Jepson Bolton Laboratory Equipment, Watford, United Kingdom) durchgeführt. Für die PCR wurde Pfu Plus Polymerase (Roboklon, Berlin, Deutschland) nach Angaben des Herstellers und folgendes Temperaturprogramm verwendet: Nach einer fünfminütigen Denaturierung bei 95°C wurden 30 Zyklen von 1 min 95°C, 1 min 60°C, 3 min 72°C gefahren sowie abschließend 7 min 72°C. Die PCR Produkte wurden über ein Agarosegel aufgetrennt, aufgereinigt und in einen TOPO/pET101 Vektor kloniert nach dem Protokoll des Herstellers (ChampionTM pET Directional TOPO® Expression Kit; Invitrogen, Carlsbad, CA, USA). E.coli TOP10 Zellen [F⁻ mcrA D(mrr-hsdRMSmcrBC) (F80lacZDM15) DlacX74 recAl deoR araD139 D(ara-leu)7697 galU galK rpsL (Str^{R}) endA1 nupG] (Invitrogen) wurden mit dem Konstruktgemisch transformiert und auf Agarplatten vereinzelt. Die so gewonnenen rekombinanten Einzelklone wurden separat kultiviert, die Plasmid-DNA isoliert, über Größenbestimmung bzw. Restriktions-Mapping identifiziert und als Template für die PCR-Amplifikation der PLE Sequenzen herangezogen. Die amplifizierten Sequenzen wurden anschließend sequenziert (MWG-Biotech, Martinsried, Deutschland).

### Konstruktion des Expressionssystems

Plasmid-DNA der einzelnen TOPO/pET101-PLE-Konstrukte wurde verdaut mit Ndel und Xhol nach dem Protokoll des Herstellers (New England Biolabs, Beverly, MA, USA; Promega, Madison, WI, USA) und die jeweiligen etwa 1694 bp großen Fragmente in mit Ndel und Xhol verdauten, über Agarosegel aufgereinigten pET15b Vektor (Novagen, Madison, WI, USA) eingefügt; dieser fügt dem Gen zusätzlich einen N-terminalen His-Tag an. Die Ligationsprodukte wurden zur Transformation von E.coli DH5α Stämmen (Novagen, Madison, WI, USA) [supE44ΔlacU169 (Φ80lacZΔM15) hsdR17 recA1 endA1 gyrA96 thi-1relA1] benutzt und das Plasmid über Kultivierung der transformierten Stämme vermehrt. Das Plasmid wurde aus den rekombinanten Stämmen isoliert und zur Kontrolle erneut sequenziert. Die so erhaltenen pET15b-PLE-Konstrukte wurden zur Transformation von E.coli Origami (DE 3) Stämmen [Δ(ara-leu)7697 ΔlacX74 ΔphoA PvuII phoR araD139 ahpC galE galK rpsL F'[lac⁺ lacI^{q} pro] (DE3) gor522::Tn10 trxB (Kan^{R}, Str^{R}, Tet^{R})4] (Novagen, Madison, WI, USA) verwendet, welche zuvor mit Plasmid pGro7 (Chaperone Plasmid Set, TAKARA BIO Inc., Otsu, Shiga, Japan) transformiert worden waren, das die Expression des Chaperons GroEL+GroES ermöglicht.

Details zur Expression der Schweineleberesterase unter Coexpression des Chaperonkomplexes GroEL/ES: Böttcher, D., Brüsehaber, E., Doderer. K., Bornscheuer, U.T. (2007), Functional expression of the gamma-isoenzyme of pig liver carboxyl esterase in Escherichia coli, Appl. Microbiol. Biotechnol., (2007), 73(6), 1282-1289

### Expression der rekombinanten PLE Isoenzyme in E.coli Origami unter Coexpression von Chaperonkomplexes GroEL/ES

Die Coexpression der Chaperone mit den PLE-Isoenzymen wurde in 150 ml LB Medium enthaltend 20 µg mL⁻¹ Chloramphenicol and 50 µg mL⁻¹ Ampicillin zur Plasmidselektion durchgeführt. Die Chaperon-Expression wurde sofort initiiert duch Zugabe von 1 mg mL⁻¹ L-Arabinose. Bei einem OD600-Wert von 0.5 wurde die PLE-Produktion durch Zugabe von 40 µM IPTG induziert. Nach 24 h wurden die Zellen abzentrifugiert, in 10 ml Natriumphosphatpuffer (50 mM, pH 7,5) resuspendiert und mit Ultraschall aufgeschlossen. Der Überstand nach Abzentrifugieren der Zellbruchstücke wurde für weitere Experimente eingesetzt (Zellrohextrakt). Der Proteingehalt und die Esterase-Aktivität wurden nach Bradford bzw. mit dem pNPA-Assay bestimmt.

### Native Polyacrylamidgelelektrophorese und Aktivitätsfärbung mit Fast Red

Eine Mischung enthaltend das Rohextrakt der rekombinant hergestellten PLE (5-15 µL, entsprechend 0.05-0.15 U der pNPA-Analyse) wurde mit Pufferlösung (20 % (w/v) Glycerin; 0,0025 % (w/v) Bromphenolblau in dH2O) (5-10 µL) gemischt. Aliquots davon wurden auf einem nativen Polyacrylamidgel (7.5 %) separiert. Zur Aktivitätsfärbung wurde das Gel in einer Mischung aus frisch hergestelltem α-Naphthylacetat und Fast Red inkubiert. Die Bildung eines roten Komplexes zwischen erzeugtem α-Naphthol und Fast Red zeigte die hydrolytische Aktivität der Esterase an. (Krebsfänger, N., et. al., (1998) Enzyme Microb. Technol, 22, 641 - 646). Anschließend wurde das Gel mit Coomassie Brilliant Blue angefärbt.

### Esterase Aktivität

Die Esterase Aktivität wurde spektrophotometrisch in Natriumphosphatpuffer (50 mM) mittels p-Nitrophenylacetat (10 mM gelöst in Dimethylsulfoxid) als Substrat bestimmt. Die Menge an erzeugtem p-Nitrophenol wurde bei einer Wellenlänge von 410 nm (ε= 15*10³ M⁻¹ cm⁻¹) bei RT und pH 7.5 ermittelt. 1 Unit (U) ist definiert als Menge an Enzym, die im Stande ist, 1 µM an p-Nitrophenol pro Minute unter Analysebedingungen umzusetzen (Krebsfänger, N., et. al., (1998) Enzyme Microb. Technol, 22, 641 -646).

Die Substratspezifität der Esterase wurde bei konstantem pH-Wert analysiert. Eine bekannte Menge an Esterase wurde zu einer Emulsion (20 mL) enthaltend ein Estersubstrat (5 % (v/v); Tributyrin, Ethylacetat, Triolein oder Methylbutyrat) und Gummi Arabicum (2 % (w/v)) bei 37°C gegeben. Die freigesetzte Säure wurde automatisch in einem pH-Statiometer (Schott, Mainz, Germany) mit 0.01 N NaOH gegentitriert, um einen konstanten pH-Wert von 7,5 beizubehalten.. 1 Unit (U) ist definiert als Menge an Enzym, die im Stande ist, 1 µM Säure pro Minute unter Analysebedingungen zu erzeugen.

### Stereoselektivität der enzymatischen Hydrolyse von Acetaten sekundärer Alkohole (siehe DE10258327A1)

Die Hydrolyse wurde in 1,5 ml Reaktionsgefäßen in einem Thermomixer (Thermomixer comfort Eppendorf, Hamburg, Deutschland) bei 37°C durchgeführt. Für 1 ml Substratlösung (10 mM in Natriumphosphatpuffer pH 7,5, 50 mM) wurden 0,5 U Esterase-Rohextrakt (bezogen auf den pNPA-Test) verwendet. Zum Abbruch der Reaktion wurde das Gemisch mit Dichlormethan extrahiert und die organische Phase über wasserfreiem Natriumsulfat getrocknet.

Die Bestimmung der Enantiomerenreinheit und der Umsatzes erfolgt gaschromatografisch. Die Enantioselektivität der Enzymvarianten wurde nach Chen et al. (C.S. Chen, Y. Fujimoto, G. Girdaukas, C.J. Sih, J. Am. Chem. Soc. 1982, 104, 7294.) berechnet.

Für eine detailliertere Beschreibung der Synthese der Substrate und der Retentionszeiten in der GC-Analytik siehe Musidlowska-Persson, A. and Bornscheuer, U.T.,"Substrate Specificity of the γ-isoenzyme of recombinant pig liver esterase towards acetates of secondary alcohols" J. Mol. Catal. B. Enzym. 2002, 19-20, 129-133.

### Stereoselektivität der enzymatischen Hydrolyse von cis-3,5-Diacetoxycyclopent-1-en

Das experimentelle Vorgehen war identisch zur Racematspaltung sekundärer Alkohole. Es wurden 0,5 Units (pNPA) PLE-Rohextrakt eingesetzt in 1 ml Reaktionsgemisch mit 10 mM Substrat in Natriumphosphatpuffer pH 7,5 50 mM. Die Reaktion wurde bei 37°C durchgeführt und zu den angegebenen Zeiten wurden Proben entnommen. Die Analyse des Umsatzes und der Produktenantiomerenüberschüsse erfolgte gaschromatografisch. Für die Analyse wurde eine Hydrodex®-b-3P (Heptakis-(2,6-di-O-methyl-3-O-pentyl-b-cyclodextrin (25 m, 0,25 mm) GC-Säule (Machery Nagel, Düren, Germany) verwendet in einem C-R5A Chromatopac/Integrator GC-Gerät (Shimadzu, Duisburg, Germany) verwendet. Die Retentionszeiten betrugen bei einer isothermen Auftrennung mit einer Säulentemperatur von 110 °C: cis-3,5-Diacetoxy-cyclopent-1-en: 21,8 min, 3(S)-Acetoxy-5(R)-hydroxy-cyclopent-1-en: 18,2 min, 3(R)-Acetoxy-5(S)-hydroxy-cyclopent-1-en: 16,0 min.

### Ergebnisse:

### Isolation der mRNA aus Schweineleber und RT-PCR

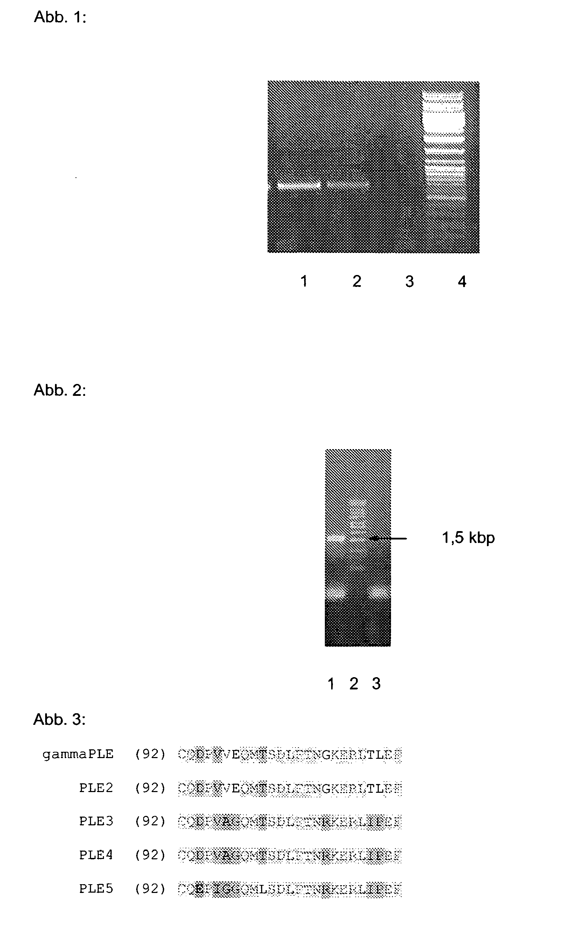

Die Qualität der isolierten RNA wurde auf einem Agarosegel überprüft (Abb. nicht gezeigt) und eine spektrophotometrische Quantifizierung vorgenommen (2,6 µg/µl); es konnten also 260 µg RNA aus 0,1 g eingesetztem Gewebe gewonnen werden. Nach dem Umschreiben in cDNA über RT-PCR wurde die Qualität der cDNA überprüft, indem sie als Template eingesetzt wurde für die Amplifikation des Haushaltsgens β-Actin. In Abb. 1 ist zu erkennen, dass die cDNA die Amplifikation des β -Actin-Gens zuließ und ihre Qualität somit geeignet für weitere Experimente ist.

Abb. 1: Kontrolle der Qualität der cDNA durch Amplifikation des Gens für β-Actin. Templates: Spur 1: humane cDNA (pos. Kontrolle), Spur 2: cDNA aus Schweineleber, Spur 3: Wasser (neg. Kontrolle).

### Amplifikation und Klonierung von PLE Genen

Die cDNA wurde zur Amplifikation von PLE Genen eingesetzt mit Primern, die auf der Sequenz der γPLE basieren. Wie in Abb. 2 zu erkennen ist, liefert die Auftragung des Reaktionsgemisches nach der PCR auf einem Agarosegel eine scharfe Bande bei etwa 1,7 kbp, was der Größe der γPLE entspricht. Diese Bande wurde ausgeschnitten, die DNA isoliert und mit Hilfe des über den forward-Primer angehängten CACC-Überhang in einen TOPO Vektor kloniert. Nach Transformation von E.coli Top10 Zellen mit diesem Konstruktgemisch wurden rekombinante Klone erhalten. Die Plasmid-DNA dieser Klone wurde isoliert, mit einem Restriktionsmapping kontrolliert und sequenziert.,

Abb. 2: Amplifikation von PLE Genen aus cDNA von Schweineleber. Spur 1: cDNA als Templat, Spur 2: 1 kbp Marker, Spur 3: Wasser als Templat (neg. Kontrolle).

### Sequenzierungsergebnisse

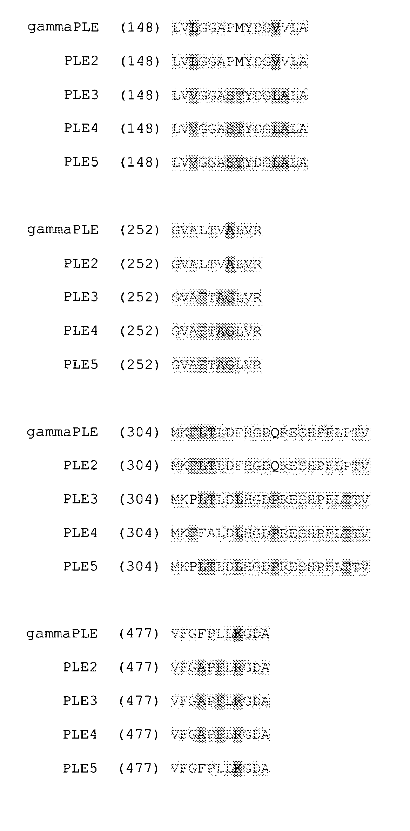

Die Sequenzierung der neuen PLE-Gene zeigte, dass vier neue, voneinander und von der γ-PLE verschiedene Gensequenzen erhalten wurden. Die von der Sequenz der γ-PLE abweichenden Stellen sind im Alignment der Aminosäuresequenzen in Abb. 3 dargestellt. Die damit vorliegenden PLEs bzw. ihre Gene wurden von 1 bis 5 durchnummeriert, wobei PLE 1 der bereits bekannten γ-PLE entspricht. Die Abweichungen der neuen PLEs gegenüber der bekannten γ-PLE lassen sich wie folgt zusammenfassen:
PLE 2: 6 Nukleotidaustausche (3 Aminosäureaustausche) [Isoenzym 4]
PLE 3: 35 Nukleotidaustausche (20 Aminosäureaustausche) [Isoenzym 24]
PLE 4: 34 Nukleotidaustausche (20 Aminosäureaustausche) [Isoenzym 39]
PLE 5: 34 Nukleotidaustausche (21 Aminosäureaustausche) [Isoenzym 41]

Abb. 3: Partielles Alignment der Aminosäuresequenzen der gefundenen PLEs 2, 3, 4 und 5 mit der Sequenz der γ-PLE (PLE 1).

### Expression der Proteine

Die fünf gefundenen Gene wurden in einen pET15b Vektor umkloniert. E.coli Origami wurde mit diesen Konstrukten und mit dem Plasmid pGro7 transformiert, welches für die beiden Chaperonteile GroES und GroEL kodiert. Für dieses Expressionssystem war in (DE 10061864) die erfolgreiche Überexpression der PLE in E. coli beschrieben worden. Die Stämme wurden in kleinem Maßstab (50 ml / 150ml) kultiviert und die Proteinexpression von Chaperon und PLE-Konstrukt induziert. Zusätzlich wurden die γPLE und ein E.coli Origami mit pGro7 ohne den pET Vektor als Vergleich mitkultiviert und die Proteinexpression induziert. Die Überstände wurden aufgeschlossen (3 ml / 9 ml Volumen) und durch Abzentrifugieren die Rohextrakte mit den löslichen intrazellulären Proteinen gewonnen, welche für folgende Experimente eingesetzt wurden.

Der Proteingehalt betrug in allen Rohextrakten etwa 7 -9 mg/ml (wovon ein Großteil des gebildeten Proteins den Chaperonen entspricht); das Endvolumen des Rohextraktes 3 bzw. 9 ml je nach Größe der Kultivierung.

### Natives Gel und Fast Red Staining

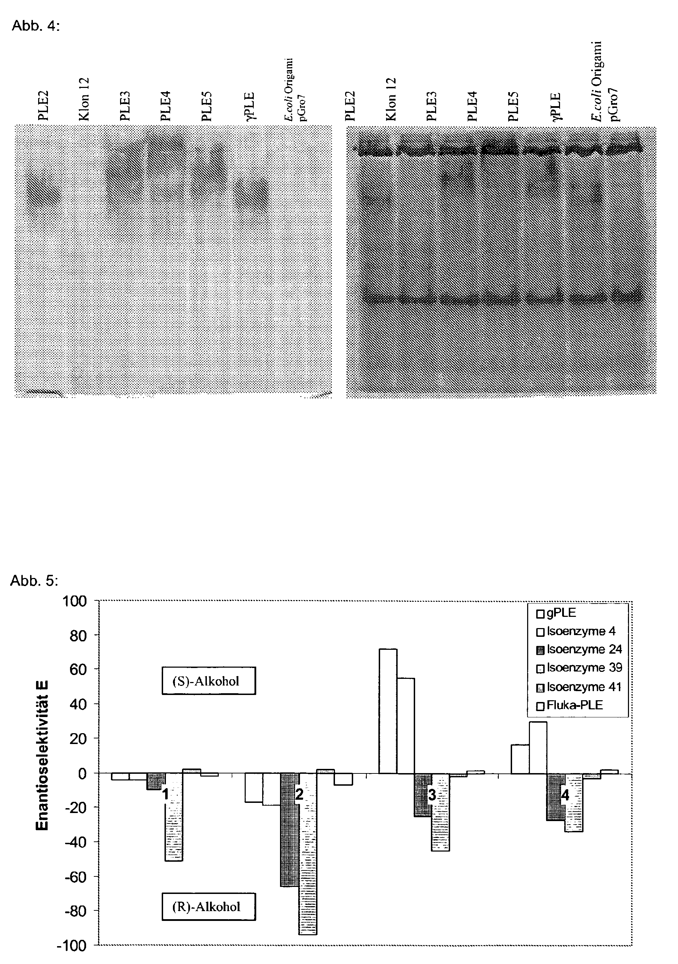

Zur Kontrolle der Esteraseaktivität wurden die Rohextrakte auf ein natives Gel aufgetragen, welches anschließend in Fast Red Lösung mit α-Naphthylacetat als Substrat inkubiert wurde. Anschließend wurde eine Coomassiefärbung durchgeführt (Abb. 4: Native PAGE der Rohextrakte von PLE2, Klon 12, PLE3, PLE4, PLE5, γPLE und dem E.coli Origami pGro7 Wildtyp (neg. Kontrolle). Links: Fast Red Staining mit α-Naphthylacetat, rechts: anschließende Coomassie Brilliant Blue Färbung.).

Man erkennt bei den PLE2, PLE3, PLE4, PLE5 und der γPLE aktive Esterase-Banden, die seltsamerweise unterschiedliche Größen aufweisen und sehr stark verschmiert sind. Da native Gele nicht so sauber laufen wie denaturierende SDS Gele, kann dies auf die Methode zurückzuführen sein; möglich ist aber auch, dass in einigen Rohextrakten sowohl trimere als auch tetramere PLE-Konstrukte vorliegen, die beide aktiv sind.

Klon 12 zeigt keine Aktivität. Im Rohextrakt des E.coli Origami ohne das pET15b Konstrukt ist wie erwartet ebenfalls keine Esterase-Aktivität nachweisbar.

Anhand der Proteinbanden in der Coomassiefärbung ist deutlich zu erkennen, dass neben der γPLE sehr viel Chaperon überexprimiert wurden. Die über Bradford bestimmten Proteingehalte umfassen also vor allem das Chaperon und sagen nichts über den Gehalt an γPLE aus. Anhand der nach der Fast Red vorgenommenen Coomassie Färbung kann keine genaue Aussage mehr getroffen werden über die Stärke der Überexpression. Man erkennt im Bereich der aktiven Banden keine zusätzliche Proteinbande im Vergleich zum E. coli Wildtyp mit Chaperon. Es kommt aber häufig vor, dass sich das Protein nach der Aktivitätsfärbung nicht mehr mit Coomassie anfärben lässt. Es könnte aber auch bedeuten, dass die Überexpression nur sehr gering ist und trotzdem Protein mit guter Aktivität liefert.

### Aktivität gegenüber p-Nitropenylacetat

Zur Überprüfung der Esterase-Aktivität wurde zunächst der pNPA-Assay verwendet. Er lieferte die Tabelle 1 aufgeführten Ergebnisse

Tab. 1. Volumenaktivitäten (U/ml) der PLE-Varianten und des Expressionsstammes (E. coli Origami mit Chaperonplasmid pGro7) gegenüber pNPA. Alle Esterasen tragen N-terminal einen His6-Tag.

| Rohextrakt | Volumenaktivität (U/ml) |
|---|---|
| PLE 1 (γ-PLE) | 11 |
| PLE 2 | 8 |
| PLE 3 | 21 |
| PLE 4 | 26 |
| PLE 5 | 40 |
| *E. coli* Origami + pGro7 | 0,02 |

Man erkennt bei allen PLEs eine Aktivität gegenüber pNPA, bei einigen der neuen Esterasen sogar eine zwei- bis vierfach höhere als bei der γ-PLE.

### Aktivität gegenüber achiralen Estern

Die Untersuchung der Aktivität der neuen PLE-Varianten gegenüber achiralen Estern wurde mit dem pH-Stat vorgenommen. Es wurden nach Aufreinigung der Enzyme spezifische Aktivitäten ermittelt, die in Tabelle 2 aufgeführt sind.

Tab. 2. Spezifische Aktivitäten der neuen PLEs und der γ-PLE gegenüber einigen achiralen Estern, ermittelt mit dem pH-Stat bei 37 °C und pH 7,5 über eine Messzeit von 10 min.

| | Tributyrin | Ethylcaprylat | Methylbutyrat | Ethylacetat | Triolein |
|---|---|---|---|---|---|
| PLE 1 (γ-PLE) | 306 | 63 | 57 | 38 | 0 |
| PLE 3 | 224 | 63 | 23 | 24 | 8 |
| PLE 4 | 131 | 44 | 76 | 25 | 9 |
| PLE 5 | 409 | 144 | 182 | 17 | 12 |

### Racematspaltung von Acetaten sekundärer Alkohole

Es wurde die Hydrolyse der folgenden racemischen Acetate untersucht:

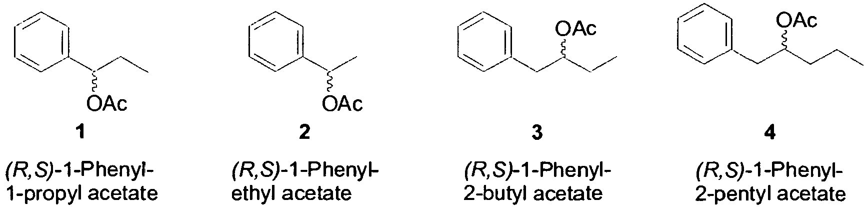

Die gaschromatographischen Untersuchungen lieferten die in Tab. 3 bis 6 gezeigten Ergebnisse. Als Vergleich wurden die von A. Musidlowska (Musidlowska-Persson, A. and Bornscheuer, U.T., J. Mol. Catal. B. Enzym. 2002, 19-20, 129-133.) ermittelten Messwerte für die kommerziellen PLE-Präparate hinzugefügt.

**Tab. 3. Enantioselektivität der neuen PLE-Varianten bei der kinetischen Racematspaltung von 1 (R,S)-1-Phenyl-1-propylacetat. (*) Messdaten für die Fluka-PLE und Chirazyme E2 wurden übernommen aus. Musidlowska-Persson, A. and Bornscheuer, U.T., J. Mol. Catal. B. Enzym. 2002, 19-20, 129-133..**

| PLE-Isoenzym | Zeit [h] | Enantiomerenüberschuss | | Umsatz [%] | E | Präferenz |
|---|---|---|---|---|---|---|
| | | [% ee_{S}] | [% ee_{P}] | | | |
| PLE 1 (γ-PLE) | 4 | 41 | 45 | 48 | 4 | R |
| PLE 2 | 2 | 38 | 49 | 44 | 4 | R |
| PLE 3 | 1 | 25 | 34 | 43 | 3 | S |
| PLE 4 | 1,5 | 51 | 71 | 42 | 10 | R |
| PLE 5 | 1 | 61 | 93 | 40 | 51 | R |
| Fluka-PLE (*) | 1 | 21 | 28 | 43 | 2,2 | R |
| Chirazyme E2 (*) | 0,5 | 18 | 27 | 40 | 2,1 | R |

**Tab..4 Enantioselektivität der neuen PLE-Varianten bei der kinetischen Racematspaltung von 2 (R,S)-1-Phenyl-ethylacetat. (*) Messdaten für die Fluka-PLE und Chirazyme E2 wurden übernommen aus. Musidlowska-Persson, A. and Bornscheuer, U.T., J. Mol. Catal. B. Enzym. 2002, 19-20, 129-133.**

| PLE-Isoenzym | Zeit [h] | Enantiomerenüberschuss | | Umsatz [%] | E | Präferenz |
|---|---|---|---|---|---|---|
| | | [% ee_{S}] | [% ee_{P}] | | | |
| PLE 1 (γ-PLE) | 2 | 74 | 77 | 49 | 17 | R |
| PLE 2 | 2 | 67 | 81 | 45 | 19 | R |
| PLE 3 | 1,5 | 18 | 24 | 43 | 2 | S |
| PLE 4 | 3 | 68 | 94 | 42 | 66 | R |
| PLE 5 | 2 | 79 | 95 | 45 | 94 | R |
| Fluka-PLE (*) | 1,5 | 65 | 56 | 54 | 7 | R |
| Chirazyme E2 (*) | 1 | 61 | 56 | 52 | 7 | R |

**Tab. 5 Enantioselektivität der neuen PLE-Varianten bei der kinetischen Racematspaltung von 3 (R,S)-1-Phenyl-2-butylacetat. (*) Messdaten für die Fluka-PLE und Chirazyme E2 wurden übernommen aus. Musidlowska-Persson, A. and Bornscheuer, U.T., J. Mol. Catal. B. Enzym. 2002,19-20, 129-133.**

| PLE-Isoenzym | Zeit [h] | Enantiomerenüberschuss | | Umsatz [%] | E | Präferenz |
|---|---|---|---|---|---|---|
| | | [% ee_{S}] | [% ee_{P}] | | | |
| PLE 1 (γ-PLE) | 4 | 83 | 93 | 47 | 72 | S |
| PLE 2 | 4 | 67 | 93 | 42 | 55 | S |
| PLE 3 | 4 | 26 | 32 | 45 | 2 | R |
| PLE 4 | 3 | 65 | 83 | 43 | 25 | R |
| PLE 5 | 4 | 82 | 89 | 48 | 45 | R |
| Fluka-PLE (*) | 2 | 12 | 12 | 49 | 1,4 | S |
| Chirazyme E2 (*) | 1 | 58 | 40 | 59 | 4 | S |

**Tab. 6.Enantioselektivität der neuen PLE-Varianten bei der kinetischen Racematspaltung von 4 (R,S)-1-Phenyl-2-pentylacetat. (*) Messdaten für die Fluka-PLE und Chirazyme E2 wurden übernommen aus. Musidlowska-Persson, A. and Bornscheuer, U.T., J. Mol. Catal. B. Enzym. 2002, 19-20, 129-133.**

| PLE-Isoenzym | Zeit [h] | Enantiomerenüberschuss | | Umsatz [%] | E | Präferenz |
|---|---|---|---|---|---|---|
| | | [% ee_{S}] | [% ee_{P}] | | | |
| PLE 1 (γ-PLE) (*) | 2 | 69 | 78 | 47 | 17 | S |
| PLE 2 | 0,2 | 71 | 87 | 45 | 30 | S |
| PLE 3 | 0,05 | 23 | 37 | 38 | 3 | R |
| PLE 4 | 0,2 | 76 | 84 | 48 | 27 | R |
| PLE 5 | 0,5 | 86 | 85 | 50 | 34 | R |
| Fluka-PLE (*) | 0,3 | 24 | 26 | 48 | 2,1 | S |
| Chirazyme E2 (*) | 0,3 | 21 | 24 | 46 | 2 | S |

In Abb. 5 sind die Unterschiede in den Enantiomerenüberschüssen noch einmal grafisch veranschaulicht.

Abb. 5. Enantioselektivitäten verschiedener Schweineleber-Esterase-Isoenzyme bei der kinetischen Racematspaltung der Substrate 1-4. Die Werte für die kommerzielle Fluka-PLE wurden aus Literatur (Musidlowska-Persson, A. and Bornscheuer, U.T., J. Mol. Catal. B. Enzym. 2002, 19-20, 129-133) übernommen.

### Hydrolyse von cis-3,5-Diacetoxycyclopent-1-en

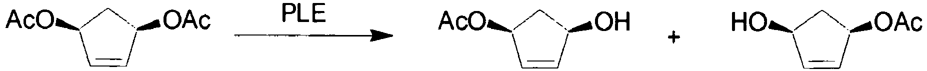

**Tab. 7. Asymmetrisierung des meso-cis-3,5-Diacetoxycyclopent-1-en durch die neuen PLE-Varianten, die γ-PLE (PLE 1) und die kommerziell erhältliche Fluka-PLE. Die Reaktion wurde mit 0,5 Units (pNPA) Rohextrakt bei 37 °C durchgeführt und die Enantiomerenüberschüsse gaschromatografisch ermittelt.**

| PLE-Isoenzym | Zeit [h] | Enantiomerenüberschuss [% ee_{P}] | Umsatz [%] | Präferenz |
|---|---|---|---|---|
| PLE 1 | 14 | 82 | 96 | **6a** (3*S*,5*R*) |
| PLE 2 | 14 | 83 | 91 | **6a** (3*S*,5*R*) |
| PLE 3 | 14 | 83 | 99 | **6a** (3*S*,5*R*) |
| PLE 4 | 14 | 42 | 95 | **6b** (3*R,*5*S*) |
| PLE 5 | 14 | 17 | 100 | **6b** (3*R,*5*S*) |
| Fluka-PLE | 20 | 61 | 100 | **6a** (3*S*,5*R*) |

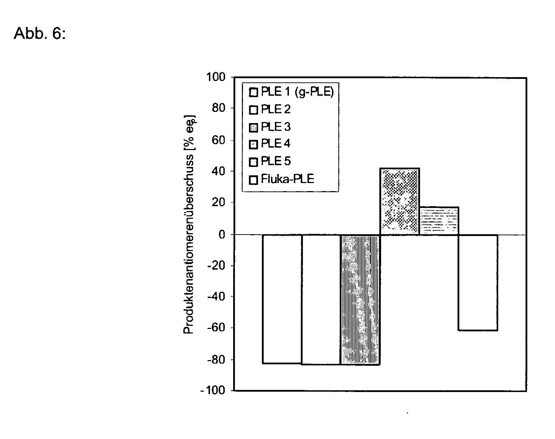

Die Unterschiede der PLE-Varianten in den Produktenantiomerenüberschüssen sind in Abb. 6 veranschaulicht.

Abb. 6. Produktenantiomerenüberschuss verschiedener Schweineleber-Esterase-Isoenzyme bei der Hydrolyse von cis-3,5-Diacetoxycyclopent-1-en.

### Einfluss von Inhibitoren

Zur Ermittlung der Hemmbarkeit der neuen PLE-Varianten wurden die Rohextrakte mit je einem von drei Esterase-Inhibitoren versetzt. Es wurde der Einfluss von Phenylmethylsulfonylfluorid, Natriumfluorid und Physostigmin untersucht. Nach bestimmten Zeitpunkten wurden Proben entnommen und die verbleibende Esterase-Aktivität mit dem pNPA-Assay bestimmt. Die Ergebnisse sind in Tab. 8 dargestellt.

**Tab. 8. Verbleibende Aktivitäten [%] der neuen PLE-Isoenzyme nach Inkubation mit den drei Inhibitoren Natriumfluorid, Phenylmethylsulfonylfluorid und Physostigmin bei 25 °C. Die Aktivitäten wurden mit dem pNPA-Assay bestimmt.**

| Inhibitor | | Konz. | Restaktivität in % | | | | |
|---|---|---|---|---|---|---|---|
| | | | PLE 1 (γ-PLE) | PLE 2 | PLE 3 | PLE 4 | PLE 5 |
| NaF | | 1 mM | | | | | |
| | 5 min | | 20 | 17 | 44 | 64 | 83 |
| | 30 min | | 21 | 15 | 46 | 66 | 88 |

| Phenylmethylsulfonylfluorid (PMSF) | | 0,01 mM | | | | | |
|---|---|---|---|---|---|---|---|
| | 1 min | | 97 | 87 | 77 | 78 | 88 |
| | 5 min | | 85 | 82 | 51 | 56 | 76 |
| | 30 min | | 55 | 50 | 7 | 7 | 24 |
| | 60 min | | 46 | 36 | 5 | 2 | 3 |

| Physostigmin | | 0,01 mM | | | | | |
|---|---|---|---|---|---|---|---|
| | 1 min | | 41 | 61 | 83 | 90 | 94 |
| | 5 min | | 12 | 25 | 81 | 82 | 98 |
| | 30 min | | 7 | 6 | 72 | 66 | 75 |

## Patentansprüche

1. Esterasen gemäß Seq. ID Nr. 6, 8 oder 10.

2. Isolierte Nukleinsäure codierend für eine Esterase gemäß Anspruch 1.

3. Gen, Vektor, Plasmid sowie rec-Mikroorganismus aufweisend eine klonierte Nukleinsäure gemäß Anspruch 2.

4. rec-Mikroorganismus nach Anspruch 3, der zusätzlich mindestens ein kloniertes Chaperon-Gen enthält.

5. Verwendung der Esterase aus Anspruch 1 zur Herstellung enantiomerenangereicherter Alkohole, Carbonsäuren bzw. Ester.

6. Verwendung der Nukleinsäuren nach Anspruch 2 in einem Verfahren zur Herstellung eines Polypeptids mit gegenüber dem Polypeptid der SEQ. ID. NO: 2 verbesserter Aktivität und/oder Selektivität und/oder Stabilität, hergestellt durch
i) Mutagenese der SEQ. ID. NO: 5, 7 oder 9,
ii) Klonierung der aus i) erhältlichen Nukleinsäuresequenz in einen geeigneten Vektor mit anschließender Transformation in ein geeignetes Expressionsystem und
iii) Detektion und Isolierung des maßgeblichen Polypeptids mit verbesserter Aktivität und/oder Selektivität und/oder Stabilität.

## Claims

1. Esterases according to SEQ ID NO: 6, 8 or 10.

2. Isolated nucleic acid encoding an esterase according to claim 1.

3. Gene, vector, plasmid and rec microorganism having a cloned nucleic acid according to claim 2.

4. The rec microorganism of claim 3, additionally comprising at least one cloned chaperon gene.

5. Use of the esterase of claim 1 for the production of enantiomer-enriched alcohols, carboxylic acids and esters, respectively.

6. Use of the nucleic acids of claim 2 in a method for the production of a polypeptide with enhanced activity and/or selectivity and/or stability relative to the polypeptide of SEQ ID NO:2, produced by
i) mutagenesis of SEQ ID NO: 5, 7 or 9,
ii) cloning of the nucleic acid sequence obtained by i) into a suitable vector with subsequent transformation into a suitable expression system and
iii) detection and isolation of the relevant polypeptide with enhanced activity and/or selectivity and/or stability.

## Revendications

1. Estérases selon la Séq. ID N°6, 8 ou 10.

2. Acide nucléique isolé codant pour une estérase selon la revendication 1.

3. Gène, vecteur, plasmide et microorganisme recombinant présentant un acide nucléique cloné selon la revendication 2.

4. Microorganisme recombinant selon la revendication 3, qui comprend en plus au moins un gène chaperon cloné.

5. Utilisation de l'estérase de la revendication 1 pour la fabrication d'alcools, acides carboxyliques ou esters énantiomériquement enrichis.

6. Utilisation des acides nucléiques selon la revendication 2 dans un procédé pour la préparation d'un polypeptide ayant une activité et/ou sélectivité et/ou stabilité améliorées par rapport au polypeptide de la SEQ ID N°2, produit par
i) mutagenèse de la SEQ ID N°5, 7 ou 9,
ii) clonage de la séquence d'acides nucléiques pouvant être obtenue au i) dans un vecteur approprié avec transformation ultérieure dans un système d'expression approprié et
iii) détection et isolation du polypeptide déterminant ayant une activité et/ou sélectivité et/ou stabilité améliorées.
